# EUROPEAN PATENT APPLICATION

(11) **EP 4 389 114 A1**
(43) Date of publication of application: **26.06.2024**
(21) Application number: 22857878.7
(22) Date of filing: 18.08.2022
(51) Int. Cl.: A61K 9/06, A61K 31/519, C07D 471/04, C07D 487/00, A61P 17/00, A61P 17/06, A61P 17/14

(54) **LOCAL TOPICAL FORMULATION CONTAINING JAK INHIBITOR OR SALT THEREOF OR CRYSTAL FORM THEREOF, PREPARATION METHOD THEREFOR AND APPLICATION THEREOF**

(30) Priority: 19.08.2021 CN 202110942954
(71) Applicant: Zhuhai United Laboratories Co., Ltd., Zhuhai, Guangdong 519040 (CN)
(72) Inventor: SHEN, Meiyue, Zhongshan, Guangdong 528467 (CN); MU, Liwei, Zhongshan, Guangdong 528467 (CN); YU, Tingting, Zhongshan, Guangdong 528467 (CN); HE, Yujun, Zhongshan, Guangdong 528467 (CN); SU, Chong, Zhongshan, Guangdong 528467 (CN); GAO, Peng, Zhongshan, Guangdong 528467 (CN); WANG, Zheng, Zhongshan, Guangdong 528467 (CN); WANG, Degang, Zhongshan, Guangdong 528467 (CN)
(74) Representative: Kröncke, Rolf
(86) International application number: PCT/CN2022/113223
(87) International publication number: WO 2023/020567

(57) **Abstract**

The present application relates to the field of pharmaceutical preparations and relates to [1,2,4] triazolo [1,5-a] pyridine compounds and their isomers or pharmaceutically acceptable salts or their crystal forms, as well as local topical preparations and their applications in skin diseases. Specifically, it relates to a local topical formulations of compound (S)-N-(5-(2-(2,2-difluorocyclopropanecarbonyl)-2-nitrospiro[3.5]non-7-yl)-[1,2,4-triazole[1,5-a]pyridine-2-yl) cyclopropaneformamide and its isomers or pharmaceutically acceptable salts or crystal forms, and their applications in skin diseases. The preferred skin diseases include psoriasis, atopic dermatitis, vitiligo, lupus erythematosus, alopecia areata, eczema, contact dermatitis, urticaria, pompholyxs, dermatomyositis, scleroderma, acne, and seborrheic dermatitis.

## Description

### Technical Field

The present application belongs to the field of pharmaceutical preparations and relates to a local topical preparation of [1,2,4] triazolo [1,5-a] pyridine compounds and isomers or pharmaceutically acceptable salts or crystal forms thereof and use thereof in skin diseases.

### Background

In recent years, JAK kinase inhibitors have become new targets for treating skin diseases. JAK kinase is a family of intracellular non receptor tyrosine kinases, consisting of four family members: JAK1, JAK2, JAK3, and TYK2. Among them, JAK1, JAK2, and TYK2 are expressed in various tissues and cells of the human body, while JAK3 is mainly expressed in various hematopoietic tissue cells. JAK inhibitors participate in many important biological processes such as cell proliferation, differentiation, apoptosis, and immune regulation by inhibiting the JAK-STAT signaling pathway. JAK inhibitors have the advantages of definite therapeutic effects and relatively small side effects, and local topical formulations can be used to treat skin related diseases (such as psoriasis, atopic dermatitis, vitiligo, lupus erythematosus, alopecia areata, etc.). The local topical administration will further reduce side effects, improve safety, and more quickly alleviate symptoms or/or solve potential causes of diseases. Local drug formulations are a significant advancement in this field.

WO/2020/038457 discloses a series of JAK kinase inhibitors of [1,2,4] triazolo [1,5-a] pyridine compounds and their isomers or pharmaceutically acceptable salts that satisfy the general formula of formula I:

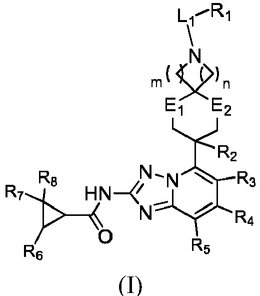

It is known that it is a small molecule JAK kinase inhibitor with significant JAK kinase inhibitory activity and high selectivity, but the local topical formulation of the JAK kinase inhibitor and its therapeutic effect on skin diseases have not been disclosed yet.

Among them, these compounds include the compounds shown in formula II, with chemical name of (S)-N-(5- (2- (2,2-difluorocyclopropane carbonyl) -2-nitrospiro[3,5]non-7-yl)-[1,2,4-triazole [1,5-a] pyridine-2-yl) cyclopropane formamide:

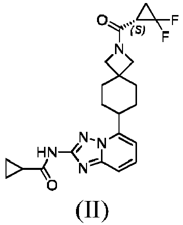

Therefore, further research is needed to discover the efficacy and safety of local topical formulations of the aforementioned small molecule compounds for skin diseases such as psoriasis, atopic dermatitis, vitiligo, lupus erythematosus, alopecia areata, allergic eczema, contact dermatitis, urticaria, pompholyx, dermatomyositis, scleroderma, acne, seborrheic dermatitis, etc., and to develop locally applied formulations with stable production processes and quality.

### summary

A purpose of the present application is to provide a safe and effective local topical preparation, and the preparation process of the topical preparation is simple, the quality is stable, and it is suitable for industrial large-scale production.

The present application provides a local topical preparation containing a JAK inhibitor, which includes the JAK inhibitor, a matrix and an additive agent, wherein the JAK inhibitor comprises a compound of formula (I), an isomer thereof, pharmaceutically acceptable salts thereof, or crystal form A thereof 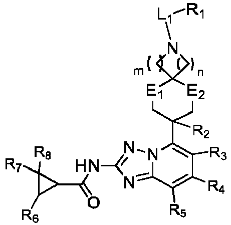 where,
E₁ and E₂ are independently selected from single bond, -CH₂- or -(CH₂)₂-, respectively;
L₁ is selected from single bond, -(CH₂)_{g}-, -c (=O)- or -c(=O)-(CH₂)ₕ-;
m is 1 or 2;
n is 1 or 2;
g is 1, 2 or 3;
h is 1, 2 or 3;
R₁ is selected from H, CN, C₁₋₆ alkyl or 3-6-membered cycloalkyl, wherein the C₁₋₆ alkyl and 3-6-membered cycloalkyl are optionally substituted by 1, 2 or 3 Rₐ;
R₂ is selected from H, F, Cl, Br, I or C₁₋₃ alkyl, wherein the C₁₋₃ alkyl is optionally substituted by 1, 2 or 3 R_{b};
R₃, R₄ and R₅ are independently selected from H, F, Cl, Br, I or C₁₋₃ alkyl, respectively, wherein the C₁₋₃ alkyl is optionally substituted by 1, 2 or 3 R_{c};
R₆, R₇ and R₈ are independently selected from H, F, Cl, Br, I or C₁₋₃ alkyl, respectively, wherein the C₁₋₃ alkyl is optionally substituted by 1, 2 or 3 R_{d};
Each Rₐ is independently selected from H, F, Cl, Br, I, CN or C₁₋₃ alkyl, respectively, wherein the C₁₋₃ alkyl is optionally substituted by 1, 2 or 3 R;
Each R_{b} is independently selected from F, Cl, Br or I;
Each R_{c} is independently selected from F, Cl, Br or I;
Each R_{d} is independently selected from F, Cl, Br or I; and
Each R is independently selected from F, Cl, Br or I.

In the present application, as one of embodiments, in the compound of formula (I), isomers thereof, or pharmaceutically acceptable salts thereof, each Rₐ is independently selected from H, F, Cl, Br, I, or CN.

In the present application, as one of embodiments, in the compound of formula (I), isomers thereof, or pharmaceutically acceptable salts thereof, R₁ is selected from H, CN, C₁₋₃ alkyl or 3-5-membered cycloalkyl, wherein the C₁₋₃ alkyl and 3-5-membered cycloalkyl are optionally substituted by 1, 2, or 3 Ra.

In the present application, as one of embodiments, in the compound of formula (I), isomers thereof, or pharmaceutically acceptable salts thereof, R₁ is selected from H, CN, CH₃, 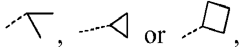 wherein CH₃, 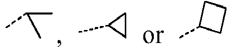 is optionally substituted by 1, 2, or 3 Ra.

In the present application, as one of embodiments, in the compound of formula (I), isomers thereof, or pharmaceutically acceptable salts thereof, R₁ is selected from H, CN, CF₃, CHF₂,

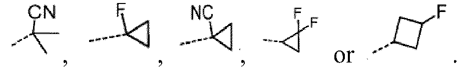

In the present application, as one of embodiments, in the compound of formula (I), isomers thereof, or pharmaceutically acceptable salts thereof, R2 is selected from H, F, Cl, Br, or I.

In the present application, as one of embodiments, in the compound of formula (I), isomers thereof, or pharmaceutically acceptable salts thereof, R₃, R₄, and R₅ are independently selected from H, F, Cl, Br, or I.

In the present application, as one of embodiments, in the compound of formula (I), isomers thereof, or pharmaceutically acceptable salts thereof, R₆, R₇, and R₈ are independently selected from H, F, Cl, Br, or I.

In the present application, as one of embodiments, in the compound of formula (I), isomers thereof, or pharmaceutically acceptable salts thereof, L₁ is selected from a single bond, -CH₂-, -(CH₂)₂-, -C(=O)- or -C(=O)-(CH₂)-.

In the present application, as one of embodiments, in the compound of formula (I), isomers thereof, or pharmaceutically acceptable salts thereof, the structural unit 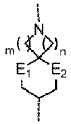 is selected from

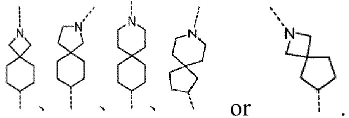

In the present application, as one of embodiments, in the compound of formula (I), isomers thereof, or pharmaceutically acceptable salts thereof, the structural unit 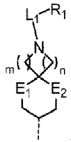 is selected from

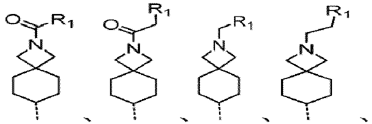

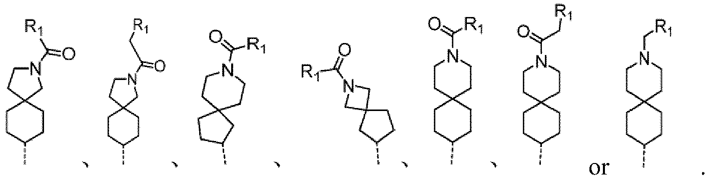

In the present application, as one of embodiments, in the compound of formula (I), isomers thereof, or pharmaceutically acceptable salts thereof, the structural unit 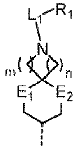 is selected from

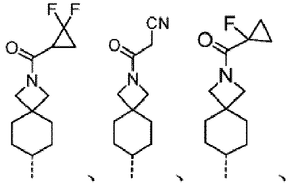

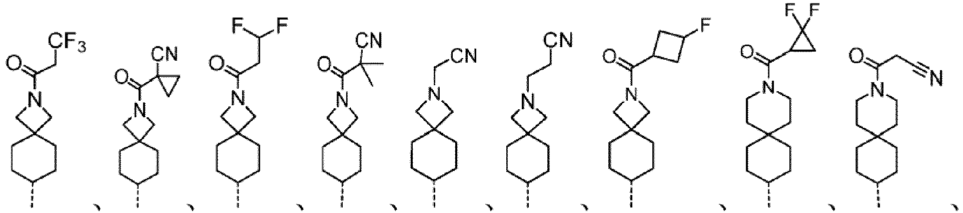

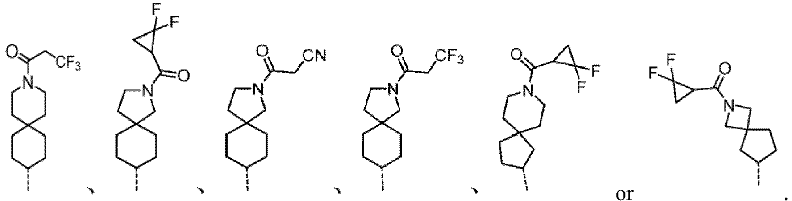

In the present application, as one of embodiments, the compound of formula (I), isomers thereof, or pharmaceutically acceptable salts thereof is selected from 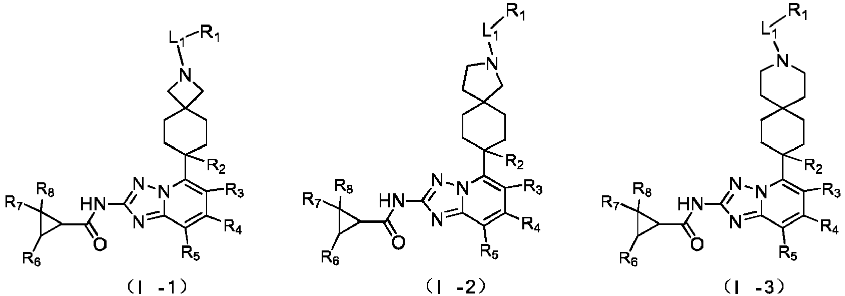 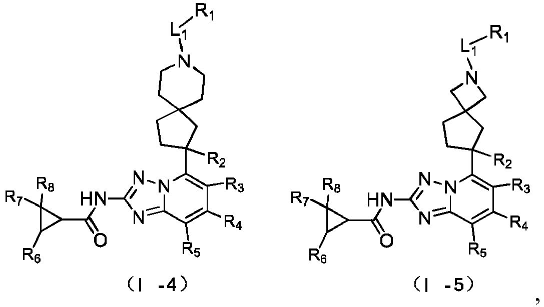 where
L₁ is defined in claim 1 or 9;
R₁ is defined in claims 1-5;
R₂ is defined in claim 1 or 6;
R₃, R₄, and R₅ is defined in claim 1 or 7; and
R₆, R₇, and R₈ is defined in claim 1 or 8.

In the present application, as one of embodiments, the compound of formula (I), isomers thereof, or pharmaceutically acceptable salts thereof is selected from 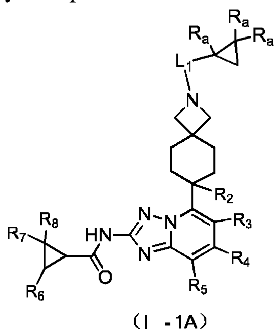 where,
L₁ is defined in claim 1 or 9;
Rₐ is defined in claim 1 or 2;
R₂ is defined in claim 1 or 6;
R₃, R₄, and R₅ are defined in claim 1 or 7;
R₆, R₇, and R₈ are defined in claim 1 or 8.

In the present application, as one of embodiments, the JAK inhibitor comprises the following compounds, their isomers or pharmaceutically acceptable salts thereof

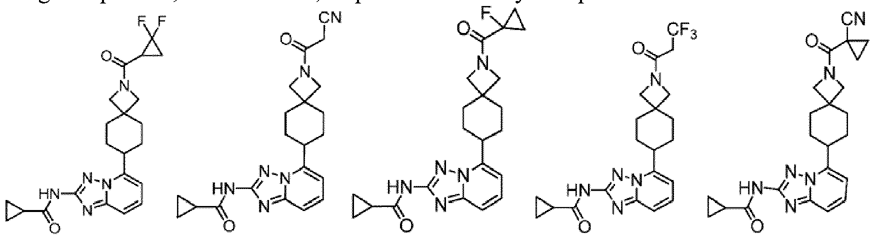

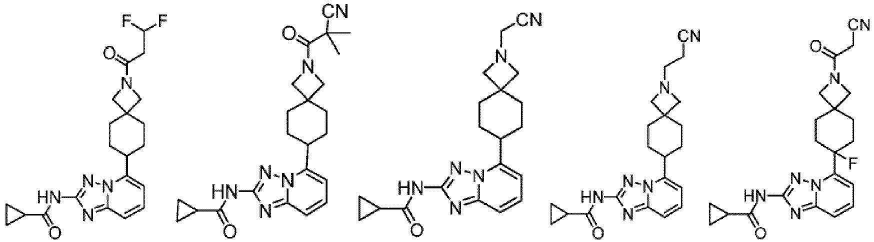

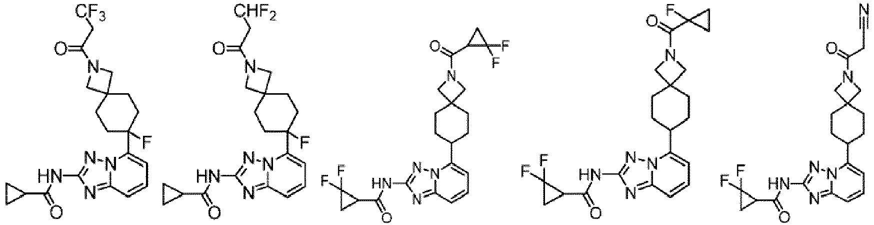

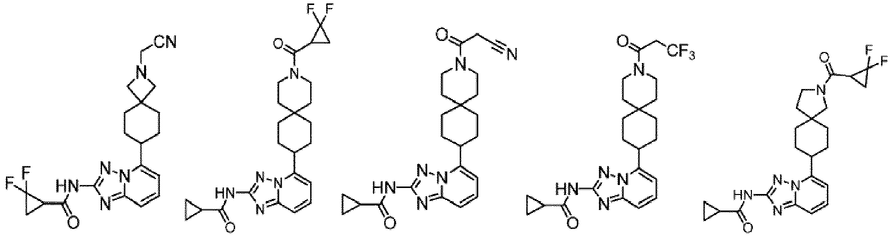

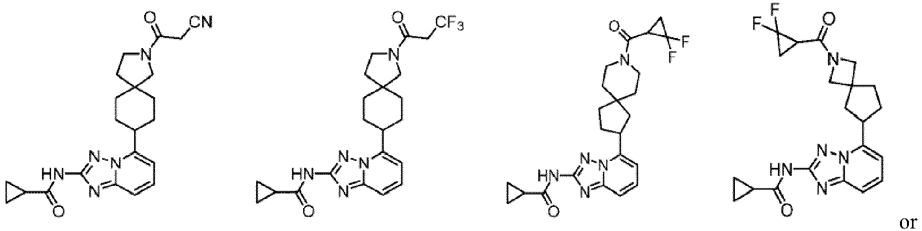

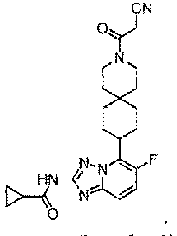

In the present application, as one of embodiments, the compound of formula (1), isomers thereof, or pharmaceutically acceptable salts thereof is selected from

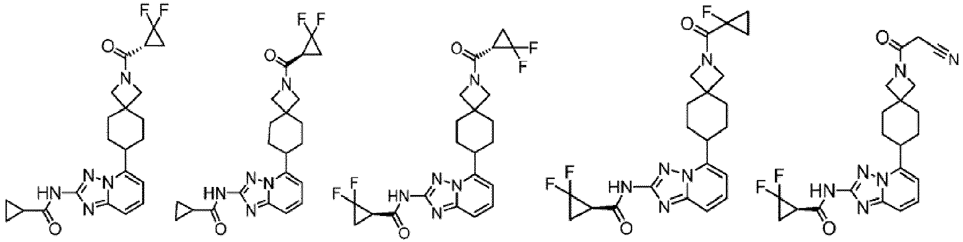

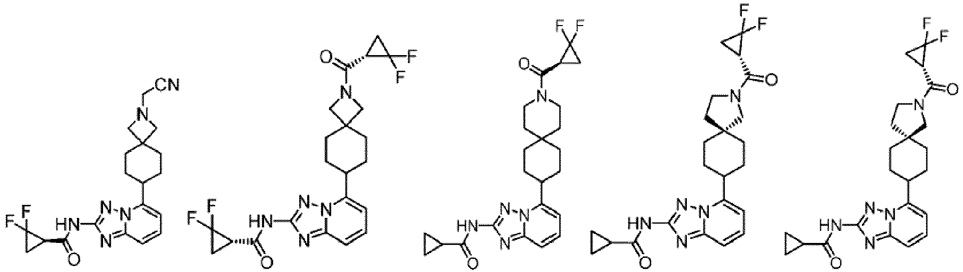

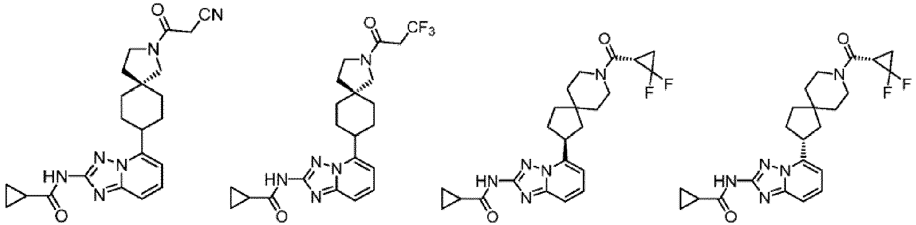

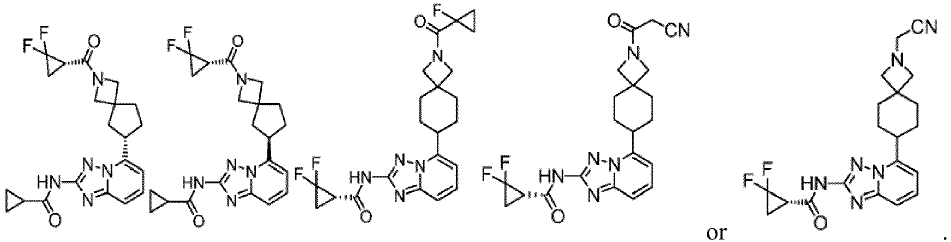

In the present application, as one of embodiments, the compound of formula (II), isomers, or pharmaceutically acceptable salts thereof:

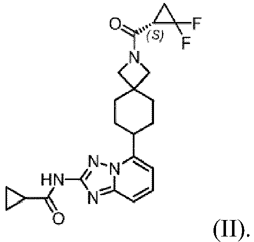

In the present application, as one of embodiments, the compound of formula (III), isomers, or pharmaceutically acceptable salts thereof:

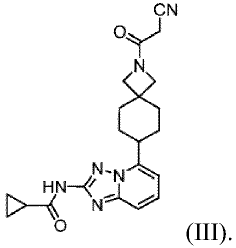

In the present application, as one of embodiments, the compound of formula (IV), isomers, or pharmaceutically acceptable salts thereof:

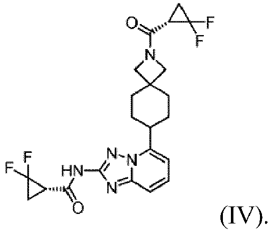

In the present application, as one of embodiments, the compound of formula (V), isomers, or pharmaceutically acceptable salts thereof:

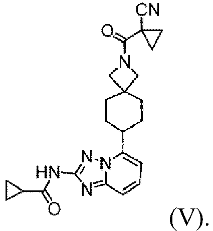

In the present application, as one of embodiments, the compound of formula (VI), isomers, or pharmaceutically acceptable salts thereof:

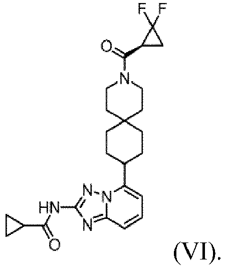

In the present application, as one of embodiments, the topical formulation includes one of the compounds of formula (II), formula (III), formula (IV), formula (V), or formula (VI) disclosed in the present application.

In the present application, as one of embodiments, the local topical preparation includes one or more pharmaceutically acceptable matrices.

In the present application, as one of embodiments, the local topical preparation includes one or more additive agents.

In the present application, as one of embodiments, the local topical formulation includes one or more matrices and one or more additives agents.

In the present application, as one of embodiments, the matrix is selected from an oil soluble matrix, or a water-soluble matrix.

In the present application, as one of embodiments, the water-soluble matrix is selected from polyethylene glycol 3350 (PEG 3350), polyethylene glycol 400 (PEG 400), cellulose derivatives, or two or more combinations thereof.

In the present application, as one of embodiments, the oil soluble matrix is selected from oil substances, hydrocarbons, lipids, synthetic (semi synthetic) oil substances, or a combination of two or more of them.

In the present application, as one of embodiments, the oil substances are selected from porcine fat, sheep fat, cow fat, sesame oil, sesame oil, cottonseed oil, soybean oil, peanut oil, olive oil, vegetable oil, hydrogenated vegetable oil, or a combination of two or more of them.

In the present application, as one of embodiments, the hydrocarbons are selected from white vaseline(also named white vaselin), yellow Vaseline (also named yellow vaselin), liquid paraffin(also named mineral oil, or paraffinum liquidum), white wax(also named white beeswax, or cera alba), paraffin, microcrystalline paraffin, ground wax, Sasol paraffin, or a combination of two or more of them.

In the present application, as one of embodiments, the lipids are selected from lanolin, beeswax, whale wax, or a combination of two or more of them.

In the present application, as one of embodiments, the synthesized (semi synthetic) oil substances are selected from squalane, lanolin derivatives, lanolin alcohol, acetylated lanolin, polyoxyethylene lanolin, hydrogenated lanolin, silicone, glyceryl behenate, palmitic acid, stearic acid, isostearic acid, octadecanol, cetanol, stearanol, or a combination of two or more of them.

In the present application, as one of embodiments, the additive includes one or more antioxidants, preservatives, moisturizers, solubilizers, or a combination of two or more of them.

In the present application, as one of embodiments, the antioxidants are selected from water-soluble antioxidants, oil soluble antioxidants, metal ion complexing agents, or a combination of two or more of them.

In the present application, as one of embodiments, the water-soluble antioxidant is selected from sodium bisulfite, sodium metabisulfite, sodium thiosulfate, sodium sulfite, vitamin C, cysteine, methionine, or a combination of two or more of them.

In the present application, as one of embodiments, the oil soluble antioxidant is selected from tert-butyl p-hydroxyanisole, dibutyl hydroxytoluene, propyl gallate, vitamin E, or a combination of two or more of them.

In the present application, as one of embodiments, the metal ion complex antioxidant is selected from edetic acid (EDTA), citric acid, tartaric acid, or a combination of two or more of them.

In the present application, as one of the implementation schemes, the preservative is selected from trichloro-tert-butanol, benzoic acid, sorbic acid, phenol, cresol, hydroxybenzyl methyl ester, hydroxyphenylethyl ester, benzalkonium bromide, benzalkonium chloride, or a combination of two or more of them.

In the present application, as one of embodiments, the moisturizing agent is selected from polyols such as glycerol, propylene glycol, sorbitol, or a combination of two or more of them.

In the present application, as one of embodiments, the solubilizer is selected from monoglyceryl linoleiate, diethylene glycol monoethyl ether, polyglycerol fatty acid ester, or a combination of two or more of them.

The present application provides a local topical preparation including the JAK inhibitor, one or more matrices, one or more additive agents, wherein the JAK inhibitor is a compound of structural formula (II), formula (III), formula (IV), formula (V), or formula (VI) and its isomer, or a pharmaceutically acceptable salt thereof, or one of its crystal forms:

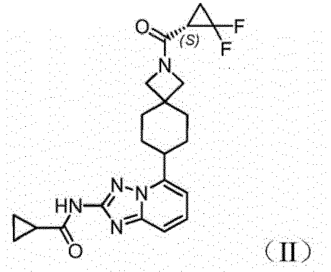

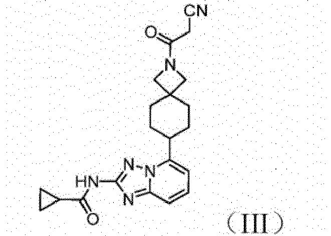

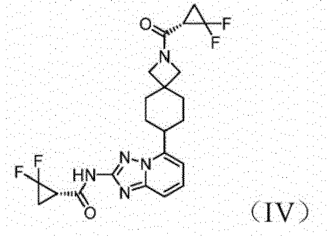

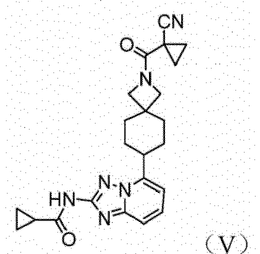

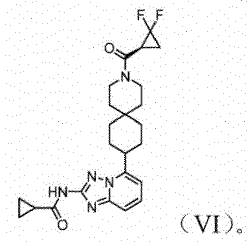

In the local topical preparation provided by the present application, a content of JAK inhibitors is 0.01% to 10%, preferably 0.02% to 9%, further preferably 0.03% to 8%, further preferably 0.04% to 7%, most preferably 0.05%, 0.06%, 0.07%, 0.08%, 0.09%, 0.1%, 0.125%, 0.2%, 0.25%, 0.3%, 0.4%, 0.5%, 0.6%, 0.7%, 0.8%, 0.9%, 1%, 1.1%, 1.2%, 1.25%, 1.3%, 1.4%, 1.5%, 1.6% 1.7%, 1.8%, 1.9%, 2%, 2.25%, 2.5%, 2.8%, 3%, 3.2%, 3.5%, 3.6%, 3.8%, 4%, 4.5%, 5%, 5.5%, 6%, or 6.5% of a total weight of the local topical preparation.

In the local topical preparation provided by the present application, the matrix may include, but not limited to PEG 3350, PEG400, cellulose derivatives, white vaseline, yellow vaseline, liquid paraffin, white wax, paraffin, ground wax, glyceryl behenate, microcrystalline paraffin, Sasol paraffin, octadecanol, stearic acid, porcine fat, lanolin, tallow, sesame oil, sesame oil, cottonseed oil, soybean oil, peanut oil, olive oil, vegetable oil, hydrogenated vegetable oil, lanolin, beeswax, whale wax, squalane, derivatives of lanolin, lanolin alcohol, acetylated lanolin, polyoxyethylene lanolin, hydrogenated lanolin, silicone, palmitic acid, stearic acid, isostearic acid, octadecanol, cetyl alcohol, stearic alcohol, etc. In the preferred embodiment of the present application, the substrate is selected from one or more of PEG 3350, PEG400, white vaseline, yellow vaseline, liquid paraffin, white wax, paraffin, ground wax, glyceryl behenate, microcrystalline paraffin, sasol paraffin, octadecanol, stearic acid, wool fat, beeswax, whale wax, squalane, octadecanol, or stearic alcohol.

In the local topical preparation provided by the present application, the additive agents may include, but not limited to one or more sodium bisulfite, sodium metabisulfite, sodium thiosulfate, sodium sulfite, vitamin C, cysteine, methionine, monoglyceride, diethylene glycol monoethyl ether, polyglycerol fatty acid ester, tert-butyl-p-hydroxyanisole, dibutylhydroxytoluene, propyl gallate, vitamin E, edelic acid (EDTA), citric acid, tartaric acid, trichloro tert butanol, benzoic acid, sorbic acid, phenol, cresol, hydroxyphenyl methyl ester, hydroxyphenyl ethyl ester, benzalkonium bromide, benzalkonium chloride, polyols, such as glycerol, propylene glycol, sorbitol.

In the present application, as one of embodiments, a content of white vaseline is 45% to 96%, preferably 50% to 95%, more preferably 53% to 94%, further preferably 55% to 93%, further preferably 56% to 92%, most preferably 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 80.5%, 81%, 81.5% 82%, 82.5%, 83%, 83.5%, 84%, 84.5%, 85%, 85.5%, 86%, 86.5%, 87%, 87.5%, 88%, 88.5%, 89%, 89.5%, 90%, 90.25%, 90.3%, 90.35%, 90.4%, 90.45%, 90.46%, 90.48%, 90.5%, 90.6%, 90.7%, 90.8%, 90.85%, 90.9%, 91%, 91.25%, 91.45%, or 91.5% of the total weight of the topical preparation.

In the present application, as one of embodiments, a content of white wax is 0.5% to 10%, preferably 1% to 9%, more preferably 2% to 8%, further preferably 3% to 6%, more preferably 4% to 5%, most preferably 4.01%, 4.02%, 4.04%, 4.06%, 4.08%, 4.1%, 4.12%, 4.14%, 4.14%, 4.16%, 4.18%, 4.2%, 4.22%, 4.24%, 4.26%, 4.28%, 4.3%, 4.32%, 4.34%, 4.36%, 4.38%, 4.4%, 4.45%, 4.5% 4.55%, 4.6%, 4.65%, 4.7%, 4.75%, 4.8%, 4.85%, 4.9%, or 4.95% of the total weight of the local topical preparation.

In the present application, as one of embodiments, a content of liquid paraffin is 0.2% to 50%, preferably 1% to 40%, more preferably 1.5% to 30%, further preferably 2% to 20%, more preferably 2.5% to 15%, most preferably 3%, 3.5%, 4%, 4.05%, 4.1%, 4.15%, 4.2%, 4.25%, 4.3%, 4.35%, 4.4%, 4.45%, 4.5%, 5%, 5.02%, 5.04%, 5.05%, 5.1%, 5.15%, 5.2%, 5.25%, 5.3% 5.35%, 5.4%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, or 14.5%. of the total weight of the local topical preparation.

In the present application, as one of embodiments, a content of paraffin is 1% to 8%, preferably 2% to 7%, most preferably 3%, 3.5%, 4%, 4.5%, 5%, 5.5%, 6%, or 6.5% of the total weight of the topical preparation.

In the present application, as one of embodiments, a content of Sasol paraffin is 1% to 10%, preferably 1.5% to 9%, more preferably 2% to 8%, further preferably 2.5% to 7%, more preferably 3% to 6%, most preferably 3.5%, 4%, 4.5%, 5%, or 5.5% of the total weight of the local topical preparation.

In the present application, as one of embodiments, the content of microcrystalline paraffin is 2% to 50%, preferably 4% to 45%, more preferably 5% to 40%, further preferably 7% to 35%, more preferably 9% to 30%, most preferably 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 20.5%, 21% 21.5%, 22%, 22.5%, 23%, 23.5%, 24%, 24.5%, 25%, 25.5%, 26%, 26.5%, 27%, 28%, or 29% of the total weight of the local topical preparation.

In the present application, as one of embodiments, the content of PEG 400 is 50% to 95%, preferably 60% to 90%, more preferably 70% to 85%, most preferably 71%, 72%, 73%, 74%, 75%, 75.2%, 75.4%, 75.6%, 75.8%, 76%, 76.2%, 76.4%, 76.6%, 77%, 77.2%, 77.4%, 77.4%, 77.6%, 77.8%, 78%, 78.2%, 78.4%, 78.6%, 78.8%, or 79% of the total weight of the local topical preparation.

In the present application, as one of embodiments, the content of PEG 3350 is 5% to 50%, preferably 8% to 40%, more preferably 10% to 30%, further preferably 15% to 25%, most preferably 16%, 16.5%, 17%, 17.5%, 18%, 18.2%, 18.4%, 18.6%, 18.8%, 19%, 19.2%, 19.4%, 19.6%, 19.8%, 20%, 20.5%, 21%, 21.5%, 22%, 22.5%, 23%, 23.5%, 24%, 24.5% of the total weight of local topical formulations.

In the present application, as one of embodiments, a content ratio of PEG 400 and PEG 3350 is 1:8-1:1, preferably 1:6-1:2, more preferably 1:5-1:3, and most preferably 1:4.

In the present application, as one of embodiments, a content of octadecanol is 1% to 10%, preferably 2% to 9%, more preferably 3% to 8%, further preferably 4% to 7%, most preferably 4.5%, 5%, 5.5%, 6%, or 6.5% of the total weight of the topical preparation.

In the present application, as one of embodiments, a content of stearic acid is 1% to 10%, preferably 1.5% to 9%, more preferably 2% to 8%, further preferably 2.5% to 7%, further preferably 3% to 6%, most preferably 3.5%, 4%, 4.5%, 5%, or 5.5% of the total weight of the topical preparation.

In the present application, as one of embodiments, a content of diethylene glycol monoethyl ether is 1% to 10%, preferably 1.5% to 9%, more preferably 2% to 8%, further preferably 2.5% to 7%, further preferably 3% to 6%, most preferably 3.5%, 4%, 4.5%, 5%, or 5.5% of the total weight of the topical preparation.

In the present application, as one of embodiments, a content of the polyglycerol fatty acid ester is 1% to 10%, preferably 1.5% to 9%, more preferably 2% to 8%, further preferably 2.5% to 7%, further preferably 3% to 6%, most preferably 3.5%, 4%, 4.5%, 5%, or 5.5% of the total weight of the local topical preparation.

In the present application, as one of embodiments, a content of monolinoleic acid glyceride is 1% to 10%, preferably 1.5% to 9%, more preferably 2% to 8%, further preferably 2.5% to 7%, further preferably 3% to 6%, most preferably 3.5%, 4%, 4.5%, 5%, or 5.5% of the total weight of the local topical preparation.

In the present application, as one of embodiments, the content of glyceryl behanate is 1% to 10%, preferably 2% to 9%, more preferably 3% to 8%, most preferably 3.5%, 4%, 4.5%, 5%, 5.5%, 6%, or 6.5% of the total weight of the local topical preparation.

In the present application, as one of embodiments, a content of monostearic acid glycerol is 1% to 10%, preferably 2% to 9%, more preferably 3% to 8%, most preferably 3.5%, 4%, 4.5%, 5%, 5.5%, 6%, 6.5%, 7%, or 7.5% of the total weight of the local topical preparation.

The local topical preparation of the present application can be prepared by methods such as grinding and melting. The local topical preparation of the present application has suitable sensory and hardness of the paste, and good compatibility with excipients.

The local topical preparation of the present application is subjected to stability testing in an environment with a temperature of 30 °C± 2 °C and a relative humidity of 65% ± 5%. After being left for one month, a HPLC method is used to determine the relevant substances, content, and observe changes in shape. The results shows that the topical preparation provided by the present application is stable in quality, and compared with 0 days after acceleration for one month, there is no significant difference in appearance, and there is no significant difference in the relevant substances and content.

The local external preparation provided by the invention can be ointment, cream, paste, gel, film, paste or patch. The local topical preparation provided by the present application is used in the preparation of drugs for treating skin diseases, and the preferred skin diseases include psoriasis, atopic dermatitis, vitiligo, lupus erythematosus, alopecia areata, eczema, contact dermatitis, urticaria, pompholyx, dermatomyositis, scleroderma, acne, and seborrheic dermatitis.

The local topical preparation provided by the present application has a high retention of active substances in the skin, which can exert better therapeutic effects in the skin. The present application creatively adopts a transdermal drug delivery method to deliver drugs, resulting in a high concentration of local active substances at the affected area and minimal transdermal absorption into the bloodstream, effectively reducing the risk of systemic drug delivery and achieving the goal of reducing toxicity and increasing efficacy.

### Definition and Explanation

Unless otherwise specified, the following terms and phrases used herein are intended to have the following meanings. A specific term or phrase should not be considered uncertain or unclear without a specific definition, but should be understood according to its ordinary meaning. When a product name appears in this article, it refers to its corresponding product or its active ingredients.

The term "pharmaceutically acceptable" used here refers to compounds, materials, compositions, and/or formulations that are within the scope of reliable medical judgment and are suitable for use in contact with human and animal tissues without excessive toxicity, irritation, allergic reactions, or other issues or complications, commensurate with a reasonable benefit/risk ratio.

The term "pharmaceutically acceptable salt" refers to the salt of a compound of the present application, prepared from a compound with a specific substituent group discovered in the present application and a relatively non-toxic acid or base. When the compounds of the present application contain relatively acidic functional groups, alkali addition salts can be obtained by contacting the neutral form of such compounds with a sufficient amount of base in a pure solution or suitable inert solvent. Pharmaceutically acceptable alkali addition salts include sodium, potassium, calcium, ammonium, organic amines or magnesium salts, or similar salts. When the compounds of the present application contain relatively basic functional groups, acid addition salts can be obtained by contacting the neutral form of such compounds with a sufficient amount of acid in a pure solution or suitable inert solvent. Examples of pharmaceutically acceptable acid addition salts include inorganic acid salts, which include, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, Phosphorous acid, etc; And organic acid salts, including acids such as acetic acid, propionic acid, isobutyric acid, maleic acid, malonic acid, benzoic acid, succinic acid, Suberic acid, fumaric acid, lactic acid, mandelic acid, phthalic acid, benzenesulfonic acid, p-toluenesulfonic acid, citric acid, tartaric acid, and methanesulfonic acid; It also includes salts of amino acids such as arginine, as well as salts of organic acids such as glucuronic acid. Certain specific compounds of the present application contain both alkaline and acidic functional groups, which can be converted into any base or acid addition salt.

The pharmaceutically acceptable salts of the present application can be synthesized by conventional chemical methods from parent compounds containing acid groups or bases. In general, the preparation method of such salts is to react these compounds in the form of free acids or bases with appropriate stoichiometric bases or acids in water, organic solvents, or a mixture of both.

The compounds of the present application may exist in specific geometric or stereoisomeric forms. The present application envisions all such compounds, including cis and trans isomers, (-)-and (+)-enantiomers, (R)-and (S)-enantiomers, diastereomers, (D)-isomers, (L)-isomers, and their racemic mixtures and other mixtures, such as enantiomers or mixtures enriched in non-enantiomer, all of which fall within the scope of the present application. Other asymmetric carbon atoms may exist in substituents such as alkyl groups. All these isomers and their mixtures are included within the scope of the present application.

Unless otherwise specified, the terms "enantiomers" or "enantiomers" refer to stereoisomers that are mirror images of each other.

Unless otherwise specified, the terms "cis trans isomer" or "geometric isomer" are caused by the inability of double bonds or single bonds of cyclic carbon atoms to rotate freely.

Unless otherwise specified, the term "diastereomeric" refers to a stereoisomer in which a molecule has two or more chiral centers and has a non mirror like relationship between molecules.

Unless otherwise specified, "(D)" or "(+)" indicates right-handed rotation, "(L)" or "(-)" indicates left-handed rotation, and "(DL)" or "(±)" indicates racemic rotation.

Unless otherwise specified, the absolute configuration of a solid center is represented by a solid wedge bond ( 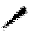 ) and a dashed wedge bond ( 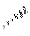 ), the relative configuration of the solid center is represented by a solid straight bond ( 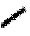 ) and a dashed straight bond ( 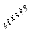 ), the solid wavy bond ( 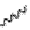 ) is represented by a solid wedge bond ( 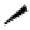 ) or a dashed wedge bond ( 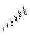 ), or the solid straight bond ( 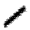 ) and the dashed straight bond ( 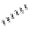 ) are represented by a wavy line ).

Unless otherwise specified, when there are double bond structures in a compound, such as carbon carbon double bonds, carbon nitrogen double bonds, and nitrogen nitrogen double bonds, and each atom on the double bond is connected to two different substituents (in double bonds containing nitrogen atoms, a pair of lone pair electrons on the nitrogen atom is considered as one of its connected substituents), if the atoms on the double bond in the compound are connected to their substituents by a wavy line ( 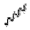 ), it represents the (Z) isomer, (E) isomer, or a mixture of two isomers of the compound. For example, the following formula (A) indicates that the compound exists in the form of a single isomer of formula (A-1) or formula (A-2), or in the form of a mixture of two isomers of formula (A-1) and formula (A-2); The following formula (B) indicates that the compound exists in the form of a single isomer of formula (B-1) or formula (B-2), or in the form of a mixture of two isomers of formula (B-1) and formula (B-2). The following formula (C) indicates that the compound exists in the form of a single isomer of formula (C-1) or formula (C-2), or in the form of a mixture of two isomers of formula (C-1) and formula (C-2).

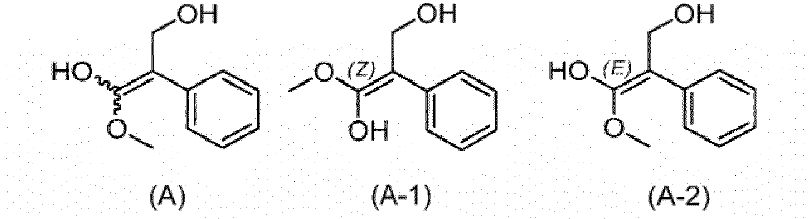

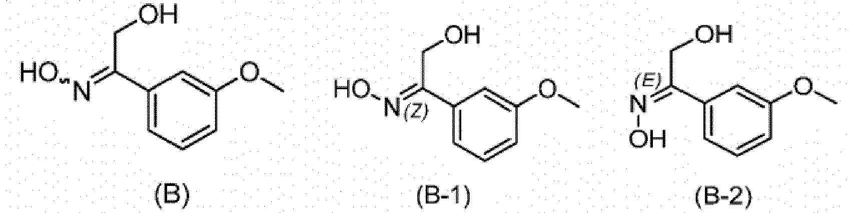

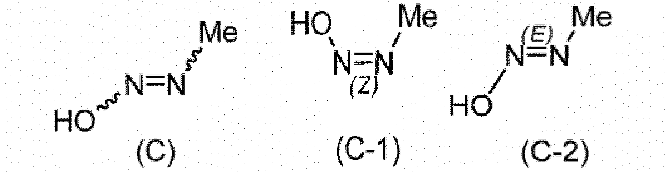

Unless otherwise specified, the terms "tautomeric" or "tautomeric form" refer to isomers of different functional groups that are in dynamic equilibrium and can quickly transform into each other at room temperature. If the tautomerism is possible (such as in solution), the chemical equilibrium of the tautomerism can be achieved. For example, proton tautomers (also known as proton transfer tautomers) include mutual transformations that occur through proton migration, such as keto-enol isomerization and imine-enamine isomerization. Valence tautomers involve the recombination of some bonding electrons for mutual conversion. The specific example of ketone enol tautomerization is the tautomerization between the two tautomers of pentane-2,4-dione and 4-hydroxypent-3-en-2-one.

Unless otherwise specified, the terms "enriched one isomer", "enriched isomer", "enriched one enantiomer", or "enantiomer enriching" refer to an isomer or enantiomer with a content of less than 100%, and the content of that isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, Or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the terms "isomer excess" or "enantiomer excess" refer to the difference between two isomers or the relative percentages of two enantiomers. For example, if one isomer or enantiomer has a content of 90% and the other isomer or enantiomer has a content of 10%, then the excess isomer or enantiomer (ee value) is 80%.

Optically active (R)-and (S)-isomers as well as D and L isomers can be prepared through chiral synthesis, chiral reagents, or other conventional techniques. If it is hopeful to obtain an enantiomer of a compound of the present application, it can be prepared by asymmetric synthesis or derivative action with chiral additives, wherein the resulting non enantiomeric mixture is separated and auxiliary groups are cleaved to provide a pure desired enantiomer. Alternatively, when the molecule contains alkaline functional groups (such as amino groups) or acidic functional groups (such as carboxyl groups), it forms non enantiomeric salts with appropriate optically active acids or bases, and then undergoes non enantiomeric separation using conventional methods known in the art, followed by recovery to obtain pure enantiomers. In addition, the separation of enantiomers and diastereomers is usually achieved through the use of chromatography, which uses chiral stationary phases and optionally combines with chemical derivatization methods (such as generating amino formates from amines). The compound of the present application may contain nonnatural proportions of atomic isotopes on one or more atoms that make up the compound. For example, compounds can be labeled with radioactive isotopes, such as tritium (³H), iodine-125 (¹²⁵I), or C-14 (¹⁴C). For example, deuterated drugs can be formed by replacing hydrogen with heavy hydrogen. The bond between deuterium and carbon is stronger than that between ordinary hydrogen and carbon. Compared to un-deuterated drugs, deuterated drugs have advantages such as reducing toxic side effects, increasing drug stability, enhancing efficacy, and prolonging drug biological half-life. The transformation of all isotopic compositions of the compound of the present application, whether radioactive or not, is included within the scope of the present application. "Optional" or "optionally" refers to events or situations that may but are not necessarily described subsequently, and the description includes the circumstances in which the event or situation occurred and the circumstances in which the event or situation did not occur.

The term "substituted" refers to any one or more hydrogen atoms on a specific atom being replaced by substituents, which can include heavy hydrogen and hydrogen variants, as long as the valence state of the specific atom is normal and the substituted compound is stable. When the substituent is oxygen (i.e.=O), it means that two hydrogen atoms are replaced. Oxygen substitution does not occur on aromatic groups. The term "optionally substituted" refers to the ability to be substituted or not to be substituted, unless otherwise specified, and the type and number of substituents can be arbitrary on the basis of chemical feasibility.

When any variable (such as R) appears more than once in the composition or structure of a compound, its definition in each case is independent. Therefore, for example, if a group is replaced by 0-2 Rs, the group can be optionally replaced by at most two Rs, and each R has an independent option. In addition, combinations of substituents and/or their variants are only allowed if such combinations produce stable compounds.

When the number of connecting groups is 0, such as-(CRR)₀-, it indicates that the connecting group is a single bond.

When one of the variables is selected from a single bond, it indicates that the two connected groups are directly connected. For example, in A-L-Z, when L represents a single bond, it indicates that the structure is actually A-Z.

When a substituent is vacant, it indicates that the substituent does not exist, for example, when X is vacant in A-X, it indicates that the structure is actually A. When the listed substituents do not indicate which atom they are connected to the substituted group, these substituents can be bonded through any of their atoms. For example, pyridine as a substituent can be connected to the substituted group through any carbon atom on the pyridine ring.

When the listed connecting groups do not indicate their connection direction, their connection direction is arbitrary. For example, if the connecting group L in 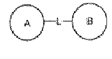 is-M-W -, then-M-W-can be connected to rings A and B to form 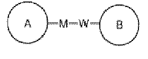 in the same direction as the reading order from left to right, or form 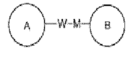 in the opposite direction as the reading order from left to right. The combination of connecting groups, substituents, and/or their variants is only allowed if such a combination produces a stable compound.

Unless otherwise specified, the number of atoms on a ring is usually defined as the number of elements in the ring. For example, a 5-7 membered ring refers to a "ring" that wraps around 5-7 atoms.

Unless otherwise specified, "5-6-membered ring" refers to a cyclic alkyl group, heterocyclic alkyl group, cyclic alkenyl group, heterocyclic alkenyl group, cyclic alkynyl group, heterocyclic alkynyl group, aryl group, or heteroaryl group composed of 5 to 6 ring atoms. The ring comprises a single ring, as well as a double ring system such as a spiral ring, a parallel ring, and a bridge ring. Unless otherwise specified, the ring may optionally contain 1, 2, or 3 independently selected heteroatoms from O, S, and N. The 5-6-membered ring includes 5-membered, 6-membered rings, etc. "5-6-membered rings" include, for example, phenyl, pyridine, and pyridine groups. On the other hand, the term "5-6-membered heterocyclic alkyl" includes pyridine groups, but does not include phenyl groups. The term "ring" also includes a ring system containing at least one ring, where each "ring" independently conforms to the above definition.

Unless otherwise specified, the term "C₁₋₆ alkyl" is used to refer to a saturated hydrocarbon group consisting of 1 to 6 carbon atoms in a straight or branched chain. The C₁₋₆ alkyl group includes C₁₋₅, C₁₋₄, C₁₋₃, C₁₋₂, C₂₋₆, C₂₋₄, C₆, and C₅ alkyl groups, etc. It can be monovalent (such as methyl), divalent (such as methylene), or multivalent (such as methyne). Examples of C₁₋₆ alkyl groups include but are not limited to methyl (Me), ethyl (Et), propyl (including n-propyl and isopropyl), butyl (including n-butyl, isobutyl, s-butyl, and t-butyl), pentyl (including n-pentyl, isopentyl, and neopentyl), hexyl, etc.

Unless otherwise specified, the term "C₁₋₃ alkyl" is used to refer to a saturated hydrocarbon group consisting of 1 to 3 carbon atoms in a straight or branched chain. The C₁₋₃ alkyl group includes C₁₋₂ and C₂₋₃ alkyl groups, etc; It can be monovalent (such as methyl), divalent (such as methylene), or multivalent (such as methyne). Examples of C₁₋₃ alkyl groups include but are not limited to methyl (Me), ethyl (Et), propyl (including n-propyl and isopropyl), etc.

Unless otherwise specified, "C₃₋₆ cycloalkyl" refers to a saturated cyclic hydrocarbon group composed of 3 to 6 carbon atoms, which is a single ring and a double ring system. The C₃₋₆ cycloalkyl group includes C₃₋₅, C₄₋₅, and C₅₋₆ cycloalkyl groups, etc; It can be monovalent, divalent, or multivalent. Examples of C₃₋₆ cycloalkyl groups include, but not limited to, cyclopropyl, cyclobutyl, cyclopentyl, cyclohexyl, etc.

Unless otherwise specified, Cₙ₋ₙ₊ₘ or Cₙ-Cₙ₊ₘ includes any specific case of n to n+m carbons, such as C₁₋₁₂ including C₁, C₂, C₃, C₄, C₅, C₆, C₇, C₈, C₉, C₁₀, C₁₁, and C₁₂, and also includes any range of n to n+m, such as C₁₋₁₂ including C₁₋₃, C₁₋₆, C₁₋₉, C₃₋₆, C₃₋₉, C₃₋₁₂, C₆₋₉, C₆₋₁₂, and C₉₋₁₂, etc; Similarly, n- membered to n+m- membered represents the number of atoms on a ring is from n to n+m. For example, a 3-12-membered ring includes 3-membered, 4-membered, 5-membered, 6-membered, 7-membered, 8-membered, 9-membered, 10-membered, 11-membered, and 12-membered rings, as well as any range from n to n+m. For example, a 3-12-membered ring includes 3-6membered, 3-9-membered, 5-6-membered, 5-7-membered, 6-7-membered, 6-8-membered, and 6-10-membered rings.

The compound of the present application can be prepared by various synthesis methods well-known to those skilled in the art, including the specific embodiments listed below, the embodiments formed by combining it with other chemical synthesis methods, and equivalent substitution methods well-known to those skilled in the art. Preferred embodiments include but not limited to embodiments of the present application.

The solvent used in the present application can be commercially available. The present application adopts the following abbreviations: aq represents water; HATU represents O-(7-azabenzotriazol-1-yl)-N,N,N',N'-tetramethylurea hexafluorophosphate; EDC represents N-(3-dimethylaminopropyl)-N'-ethylcarbodiimide hydrochloride; m-CPBA represents 3-chloroperoxybenzoic acid; Eq represents equivalent, equivalent; CDI represents carbonyl diimidazole; DCM represents dichloromethane; PE represents petroleum ether; DIAD represents diisopropyl azodicarboxylic acid; DMF represents N, N-dimethylformamide; DMSO represents dimethyl sulfoxide; EtOAc represents ethyl acetate; EtOH represents ethanol; MeOH represents methanol; CBz represents benzyloxycarbonyl, which is an amine protecting group; BOC represents tert butoxycarbonyl, which is an amine protecting group; HOAc represents acetic acid; NaCNBH3 represents sodium cyanide borohydride; r.t. represents room temperature; O/N represents overnight stay; THF represents tetrahydrofuran; Boc₂O represents ditert butyl dicarbonate; TFA represents trifluoroacetic acid; DIPEA represents diisopropylethylamine; SOCl₂ represents sulfoxide chloride; CS₂ represents carbon disulfide; TsOH represents p-toluenesulfonic acid; NFSI represents N-fluoro-N-(benzenesulfonyl) benzenesulfonamide; NCS represents 1-chloropyrrolidine-2,5-dione; N-Bu4NF represents tetrabutylammonium fluoride; IPrOH represents 2-propanol; mp represents the melting point; LDA represents lithium diisopropylamine; Pd (dppf) Cl₂ • CH₂Cl₂ represents the dichloromethane complex of [1,1'- bis (diphenylphosphine) ferrocene] palladium dichloride; EDCI represents carbodiimide; DIEA represents N, N-diisopropylethylamine; IPA represents isopropanol; HOBt represents 1-hydroxybenzotriazole; LiHMDS represents hexamethyldisilicylamine lithium; TEA represents triethylamine; HEPES represents 4-hydroxyethylpiperazine ethanesulfonic acid; LiHMDS represents hexamethyldisilicylamine lithium; EDCI represents carbodiimide; Pd/C represents palladium carbon; METHANOL represents methanol; KOAc represents potassium acetate; K₂CO₃ represents potassium carbonate.

Compound II refers to the active ingredient compound (S)-N-(5- (2-(2,2-difluorocyclopropane carbonyl)-2-nitrospiro[3.5]non-7-yl)-[1,2,4-triazole[1,5-a] pyridine-2-yl) cyclopropane formamide and its isomers or pharmaceutically acceptable salts or crystal forms (also known as "compound (II)" or "compound (II)"). Compound III in the present application refers to the isomer or pharmaceutically acceptable salt or crystal form of a compound with structural formula III. Compound IV in the present application refers to the isomer or pharmaceutically acceptable salt or crystal form of a compound with structural formula IV Compound V in the present application refers to the isomer or pharmaceutically acceptable salt or crystal form of a compound with structural formula V. Compound VI in the present application refers to the isomer or pharmaceutically acceptable salt or crystal form of a compound with structural formula VI. 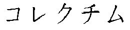 Ointment 0.5% refers to the specification of 0.5% Corecim produced in Japan ^{®} Ointment, also known as Delgocitinib ointment or Digotinib ointment.

Compounds manually or ChemDraw^{®} Software naming, using supplier catalog names for commercially available compounds.

### Brief Description of the Drawings

FIG. 1: Intradermal retention of compound II in pig skin in Examples 21-24.
FIG. 2: Changes in thickness of a right ear of rats in Experiment Example 3.
FIG. 3: Swelling degree (ear weight) of the right ear of rats in Experiment Example 3.
FIG. 4: Scores of intact skin irritation test in Experiment Example 4.
FIG. 5: Irritation test score for single administration of damaged skin in Experiment Example 4.
FIG. 6: Rheological curve of Example 21.
FIG. 7: Rheological curve of Example 22.
FIG. 8: Rheological curve of Example 23.
FIG. 9: Rheological curve of Example 24.
FIG. 10: Blood concentration-time curve of male and female Bama miniature pigs in Experiment Example 6 after a single intravenous injection of 0.2 mg/kg compound II.
FIG. 11: Blood concentration-time curve of male and female Bama miniature pigs in Experiment Example 6 after a single percutaneous administration of an ointment prepared in Example 24.

### Detailed Description

Further detailed illustration of the present application will be provided through the following examples. These examples are provided for illustrative purposes only and are not intended to limit the scope of the present application.

### Examples 1-2

Polyethylene glycol 3350 (PEG 3350), polyethylene glycol 400 (PEG 400), white vaseline, and white wax were heated and stirred in a water bath until completely melted. Prescribed amount of compound II was added to the matrix solution, stirred evenly, and quenched to obtain the preparation. The specific data is shown in Table 1.

**Table 1**

| Component (g/tube) | Examples | |
|---|---|---|
| | Example 1 | Example 2 |
| Compound II | 0.10 | 0.05 |
| White vaseline | - | 4.70 |
| White wax | - | 0.25 |
| PEG 3350 | 0.98 | - |
| PEG 400 | 3.92 | - |
| Prescription total weight | 5.00 | 5.00 |

### Examples 3-7

White vaseline, liquid paraffin, white wax, and glyceryl behenate were heated and stirred in a water bath according to the ratio in Table 2 until completely melted. Prescribed amount of compound II was added to the matrix solution, stirred evenly, and quenched to obtain the preparation.

**Table 2**

| Component (g/tube) | Examples | | | | |
|---|---|---|---|---|---|
| | 3 | 4 | 5 | 6 | 7 |
| Compound II | 0.05 | 0.05 | 0.05 | 0.05 | 0.05 |
| White Vaseline | 2.80 | 3.25 | 3.25 | 3.35 | 2.65 |
| Liquid paraffin | 2.05 | 1.55 | 1.5 | 1.30 | 2.00 |
| White wax | - | - | - | - | 0.20 |
| Glycerol behenate | 0.10 | 0.15 | 0.20 | 0.30 | 0.10 |
| Prescription total weight | 5.00 | 5.00 | 5.00 | 5.00 | 5.00 |

In Examples 3-7, the effects of the proportion of thickener Glycerol behenate at 2%, 3%, 4%, and 6% on the external sensory perception of the ointment were investigated.

Examples 5 and 6 had poor extrudability and a slightly hard paste. Example 3, Example 4, and Example 7 had good extrusion performance, and the paste was moderately soft and hard. After being kept at a constant temperature of 30 °C for 10 days, particles precipitated from the paste and the compatibility of the excipients was poor.

### Examples 8-13

White Vaseline, liquid paraffin, paraffin, white wax, microcrystalline wax, and Sasol paraffin were heated and stirred according to the ratio in Table 3 until completely melted. Prescribed amount of compound II was added to the matrix solution, stirred evenly, and quenched to obtain the preparation.

**Table 3**

| component (g/tube) | Examples | | | | | |
|---|---|---|---|---|---|---|
| | 8 | 9 | 10 | 11 | 12 | 13 |
| Compound II | 0.05 | 0.05 | 0.05 | 0.05 | 0.05 | 0.05 |
| White Vaseline | 3.25 | 2.75 | 4.50 | 4.30 | 4.50 | 4.30 |
| Liquid paraffin | 1.20 | 1.20 | 0.25 | 0.35 | 0.25 | 0.35 |
| Paraffin | - | - | 0.20 | - | - | 0.15 |
| White wax | - | - | - | 0.30 | - | 0.15 |
| Microcrystallin e wax | 0.50 | 1.00 | - | - | - | - |
| Sasol paraffin | - | - | - | - | 0.20 | - |
| Prescription total weight | 5.00 | 5.00 | 5.00 | 5.00 | 5.00 | 5.00 |

Paraffin, white wax, microcrystalline wax, and Sasol paraffin are mainly used as components of ointments for local drug prescriptions, and are used in ointments to increase melting point or hardness. The melting point of Sasol paraffin in Example 12 was very high, and it was still not possible to mix Sasol paraffin evenly with other components of the matrix at high temperatures. The ointment obtained from Examples 8, 9, 10, 11, and 13 had a moderate softness and hardness. After being placed at a constant temperature of 30 °C, oily substances precipitated and the melting point of the ointment was relatively low in Examples 8 and 9. The sensory hardness of the cream in Examples 10, 11, and 13 was acceptable, while the cream in Examples 11 and 13 was smoother and more fine. The addition of white wax can effectively regulate the hardness and texture of the ointment matrix.

### Examples 14-16

White vaseline, liquid paraffin, white wax, octadecanol, and stearic acid in the proportions shown in Table 4 were mixed, and continuously stirred in a 70 °C water bath until completely melted. Prescribed amount of compound II was added to the matrix solution, homogenized and dispersed at 70 °C, stirred and cooled to room temperature to obtain the ointment.

The paste in Examples 14, 15, and 16 showed relatively suitable sensory hardness, good extrudability, and a uniform, smooth, and fine texture.

**Table 4**

| Component (g/tube) | Examples | | | |
|---|---|---|---|---|
| | 2 | 14 | 15 | 16 |
| Compound II | 0.05 | 0.05 | 0.05 | 0.05 |
| White Vaseline | 4.70 | 4.50 | 4.50 | 4.50 |
| Liquid paraffin | - | 0.25 | 0.25 | 0.25 |
| White wax | 0.25 | - | 0.20 | - |
| Octadecyl alcohol | - | - | - | 0.25 |
| Stearic acid | - | 0.20 | - | - |
| Prescription total weight | 5.00 | 5.00 | 5.00 | 5.00 |

### Examples 17-20

White vaseline, liquid paraffin, white wax, monoglyceryl linoleic acid ester, diethylene glycol monoethyl ether, and polyglycerol fatty acid ester in the proportions shown in Table 5 were mixed, and continuously stirred in a 70 °C water bath until completely melted. Prescribed amount of compound II was added to the matrix solution, homogenized and dispersed at 70 °C, stirred and cooled to room temperature to obtain the ointment.

Example 17: When preparing the ointment, the matrix was homogenized at 500 rpm using a high shear homogenizer in a molten state. The active ingredients could not be dissolved, and the resulting ointment still had a granular texture. The matrix was not fine and smooth. The active substance in the ointment of Examples 18, 19, and 20 was not completely dissolved, and the solubilizer did not increase the solubility of the active substance. The active substance still is suspended in particle state.

**Table 5**

| Component (g/tube) | Examples | | | |
|---|---|---|---|---|
| | 17 | 18 | 19 | 20 |
| Compound II | 0.05 | 0.05 | 0.05 | 0.05 |
| White Vaseline | 4.50 | 4.50 | 4.50 | 4.50 |
| Liquid paraffin | - | 0.10 | 0.10 | 0.10 |
| White wax | 0.20 | 0.10 | 0.10 | 0.10 |
| Monooleic acid glyceride | - | - | 0.25 | - |
| Diethylene glycol monoethyl ether | 0.25 | - | - | 0.25 |
| Polyglyceryl fatty acid ester | - | 0.25 | - | - |
| Prescription total weight | 5.00 | 5.00 | 5.00 | 5.00 |

### Examples 21-24

According to the prescription proportions of Example 21, Example 22, Example 23, and Example 24 in Table 6, white vaseline, white wax, and liquid paraffin were added to a emulsifier in sequence. The cover was closed and stirring was performed at low speed of 20 rpm at a main pot temperature of 70 °C. After all the matrices were melted and stirred evenly, compound II in the prescribed proportion was added, the main pot was maintained at a temperature of 65 °C and vacuum degree of -0.08 Mpa, stirred at low speed of 40 rpm, and emulsified at high speed of 3500 rpm for 4 hours. After compound II was evenly dispersed, the paste was started to be cooled for a cooling time of 2.5 hours. During the cooling process of the paste, stirring was continued until the matrix turned white and the paste was formed. Then high-speed emulsification was stopped, and a low-speed stirring was performed at 60 rpm. During the cooling period, the low-speed stirring speed gradually decreased to 20 rpm, and the temperature dropped to around 35 °C to obtain the ointment.

**Table 6**

| Component (g/tube) | Examples | | | |
|---|---|---|---|---|
| | 21 | 22 | 23 | 24 |
| Compound II | 0.005 | 0.025 | 0.05 | 0.1 |
| White Vaseline | 4.540 | 4.523 | 4.50 | 4.45 |
| Liquid paraffin | 0.253 | 0.251 | 0.25 | 0.25 |
| White wax | 0.202 | 0.201 | 0.20 | 0.20 |
| Prescription total weight | 5.00 | 5.00 | 5.00 | 5.00 |

Examples 21 to 23 showed an off white color on the paste, while Examples 24 showed a white color on the paste. Examples 21-24 showed that the sensory viscosity of the cream was moderate, the softness and hardness were proper, and the extrudability was good. The texture was smooth and fine, and the ointment had good extensibility. It is easy to apply and retain on the skin, which is conducive to the active ingredients in local topical preparations exerting their therapeutic effects on the skin.

### Examples 25-26

White vaseline, liquid paraffin, paraffin, and monostearate glycerides were heated and stirred in a water bath according to the ratio in Table 7 until completely melted. Prescribed amount of compound II was added to the matrix solution, stirred evenly, and quenched to obtain the preparation.

**Table 7**

| Component (g/tube) | | Examples | | |
|---|---|---|---|---|
| | | 25 | | 26 |
| Compound II | 0.05 | | 0.05 | |
| Glycerol mono- and di-stearate | 0.35 | | 0.15 | |
| Paraffin | 0.25 | | - | |
| Liquid paraffin | 0.45 | | 1.30 | |
| White Vaseline | 3.90 | | 3.50 | |
| Prescription total weight | 5.00 | | 5.00 | |

Examples 25 and 26 were used to investigate the effect of adding thickener glycerol mono- and di-stearate on the external sensory perception of the ointment. Example 25 showed poor extrusion performance and hard paste. After being kept at a constant temperature of 40 °C for 5 days, particles precipitated from the paste. On the basis of Example 25, Example 26 reduced the amount of glycerol mono- and di-stearate, while removing paraffin and increasing the amount of liquid paraffin, without significantly improving the hardness of the ointment, and there were particles precipitated from the ointment. The compatibility between excipients in Examples 25 and 26 was poor.

### Examples 27-30

According to the prescription proportion of Example 27-30 in Table 8, white vaseline, white wax, and liquid paraffin were successively added to an emulsifier, covered by a lid, and stirred at a low speed of 20 rpm at a main pot temperature of 70 °C. After all the matrices were melted and stirred evenly, compound III in the prescribed proportion was added, the main pot was maintained at a temperature of 65 °C and a vacuum degree of -0.08 Mpa, stirred at a low speed of 40 rpm, and emulsified at high speed of 3500 rpm for 4 hours. After compound III was evenly dispersed, the paste was started to be cooled for a cooling time of 2.5 hours. During the cooling process of the paste, stirring was continued until the matrix turned white and became paste. High-speed emulsification was stopped, the low-speed stirring speed was adjusted to 60 rpm, and stirring continued. During the cooling period, the low-speed stirring speed gradually decreased to 20 rpm, and the temperature dropped to around 35 °C to obtain the ointment.

**Table 8**

| Component (g/tube) | Examples | | | |
|---|---|---|---|---|
| | 27 | 28 | 29 | 30 |
| Compound III | 0.005 | 0.025 | 0.05 | 0.1 |
| White Vaseline | 4.540 | 4.523 | 4.50 | 4.45 |
| Liquid paraffin | 0.253 | 0.251 | 0.25 | 0.25 |
| White wax | 0.202 | 0.201 | 0.20 | 0.20 |
| Prescription total weight | 5.00 | 5.00 | 5.00 | 5.00 |

Compounds III of Examples 27-30 were dispersed in a suspended state in an off-white matrix, and no particles of more than 180 µm were observed. The ointment had fine and smooth texture, good extensibility, good tendency to be applied and remained on the surface of skin, forming a uniform oil film.

### Examples 31-34

White vaseline, white wax, and liquid paraffin were added to the emulsifier according to the prescribed proportions in Table 9, covered by a lid, and stirred at a low speed of 20 rpm at a main pot temperature of 70 °C. After all the matrices were melted and stirred evenly, compound IV in the prescribed proportion was added, maintained at the temperature in the main pot at 65 °C, vacuum degree at -0.08 Mpa, stirred at low speed of 40 rpm, and emulsified at high speed of 3500 rpm for 4 hours. After compound IV was evenly dispersed, the paste was started to be cooled for a cooling time of 2.5 hours. During the cooling process of the paste, stirring was continued until the matrix turned white and the paste was formed. High-speed emulsification was stopped, the low-speed stirring speed was adjusted to 60 rpm, and then low-speed stirring continued. During the cooling period, the low-speed stirring speed gradually decreased to 20 rpm, and the temperature dropped to around 35 °C to obtain the ointment.

**Table 9**

| Components (g/tube) | Examples 31 | Examples 32 | Examples 33 | Examples 34 |
|---|---|---|---|---|
| Compound IV | 0.005 | 0.025 | 0.05 | 0.1 |
| White Vaseline | 4.540 | 4.523 | 4.50 | 4.45 |
| Liquid paraffin | 0.253 | 0.251 | 0.25 | 0.25 |
| White wax | 0.202 | 0.201 | 0.20 | 0.20 |
| Prescription total weight | 5.00 | 5.00 | 5.00 | 5.00 |

Examples 31-34: The active component compound IV particles were uniformly dispersed in the matrix, without particles of more than 180µm. The matrix had good extensibility and can form a uniform oil film on the skin surface, which is conducive to the efficacy of active ingredients.

### Examples 35-38

White vaseline, white wax, and liquid paraffin were added to the emulsifier in turn according to the prescription proportion in Table 10, covered by a lid, and stirred at low speed at 20 rpm at a main pot temperature of 70 °C. After all the matrix was melted and stirred evenly, compound V in the prescribed proportion, maintained at the temperature in the main pot at 65 °C, vacuum degree at -0.08 Mpa, stirred at low speed of 40 rpm, and emulsified at high speed of 3500 rpm for 4 hours. After compound V was evenly dispersed, the paste was started to be cooled for a cooling time of 2.5 hours. During the cooling process of the paste, stirring was continued until the matrix turned white and the paste was formed. High-speed emulsification was stopped, the low-speed stirring speed was adjusted to 60 rpm, and then low-speed stirring continued. During the cooling period, the low-speed stirring speed gradually decreased to 20 rpm, and the temperature dropped to around 35 °C to obtain the ointment.

**Table 10**

| Components (g/tube) | Example35 | Example36 | Example37 | Example38 |
|---|---|---|---|---|
| Compound V | 0.005 | 0.025 | 0.05 | 0.1 |
| White Vaseline | 4.540 | 4.523 | 4.50 | 4.45 |
| Liquid paraffin | 0.253 | 0.251 | 0.25 | 0.25 |
| White wax | 0.202 | 0.201 | 0.20 | 0.20 |
| Prescription total weight | 5.00 | 5.00 | 5.00 | 5.00 |

The ointment obtained from Examples 35-38 was moderately soft and hard, easy to fill, and had excellent extrusion properties. The ointment was smooth and fine, and the active ingredients can be evenly dispersed in the matrix. This oily matrix can be evenly applied to the surface of the skin, which is beneficial for the skin to retain moisture.

### Examples 39-42

White vaseline, white wax, and liquid paraffin were added to the emulsifier in turn according to the prescription proportion in Table 11, covered by a lid, and stirred at low speed at 20 rpm at a main pot temperature of 70 °C. After all the matrix was melted and stirred evenly, compound VI in the prescribed proportion was added, maintained at the temperature in the main pot at 65 °C, vacuum degree at -0.08 Mpa, stir at low speed of 40 rpm, and emulsify at high speed of 3500 rpm for 4 hours. After the dispersion of compound VI was uniform, the paste was started to be cooled for a cooling time of 2.5 hours. During the cooling process of the paste, stirring was continued until the matrix turned white and the paste was formed. High-speed emulsification was stopped, the low-speed stirring speed was adjusted to 60 rpm, and then low-speed stirring continued. During the cooling period, the low-speed stirring speed gradually decreased to 20 rpm, and the temperature dropped to around 35 °C to obtain the ointment.

**Table 11**

| Components (g/tube) | Example39 | Example40 | Example41 | Example42 |
|---|---|---|---|---|
| Compound VI | 0.005 | 0.025 | 0.05 | 0.1 |
| White Vaseline | 4.540 | 4.523 | 4.50 | 4.45 |
| Liquid paraffin | 0.253 | 0.251 | 0.25 | 0.25 |
| White wax | 0.202 | 0.201 | 0.20 | 0.20 |
| Prescription total weight | 5.00 | 5.00 | 5.00 | 5.00 |

In Examples 39-42 prepared according to the prescription proportion in Table 11, Examples 41 and 42 had a smooth, white, and glossy cream, while Examples 39 and 40 had a white and fine cream. Examples 39-42 showed no difference in extensibility and were relatively easy to apply to the skin, forming a transparent oily film that can reduce the loss of skin moisture and facilitate the active ingredients to exert their pharmacological effects.

### Experiment Example 1: In vitro transdermal test of ointment

ex vivo transdermal tests were conducted on the ointments prepared from Examples 1, 2, 14, 15, 16, 18, 19, 20, 21, 22, 23, and 24 to investigate the ability of active ingredients from different formulations to remain on the skin.

Preparation of ex vivo skin: the skin of the same part of a pig ear and back was selected as the experimental model, pig hair was removed, excess subcutaneous fat was peeled off, and the thickness of the pig skin was controlled to be between 700 and 800 µm. After checking the integrity of the skin through a microscope to ensure that there is no damage to the skin, it was rinsed thoroughly with physiological saline, placed in a sealed aluminum foil, and stored at -70 °C for later use.

In vitro transdermal experiment: the detached pig skin was restored to room temperature slowly, laid flat and fixed with clips between the two vertical diffusion pools, with the stratum corneum facing the supply pool and the dermis facing the receiving pool. They were fed with medicine using a fixed thickness aluminum plate and weighed, then placed in the supply pool. A rotor and phosphate buffer solution were added to the receiving pool (phosphate buffer solution was used as the receiving solution, which has been confirmed by preliminary research to meet the requirements of the leakage tank), so as to avoid the generation of bubbles. The temperature of the multifunctional transdermal diffusion instrument was set to 32 ± 0.1 ° C and the rotational speed was set to 600 rpm. The experiment was stopped after 5 hours and samples was taken from the receiving pool. At the same time, residual ointment from the supply pool and skin samples at the contact surface of the ointment were collected, and extracted using suitable solvents for ultrasonic extraction. The drug content of the above samples was detected by LC-MS, and the recovery rate was calculated.

The content of active ingredients in the skin was the intradermal retention amount, and the content of active ingredients in the receiving pool was the cumulative transmittance amount. The results of the in vitro transdermal test of the ointment are shown in Table 12.

**Table 12**

| Exam ples | Cumulative transmittance amount (ng) | Intradermal retention amount (ng) | Recovery rate (%) |
|---|---|---|---|
| 1 | 0.00 | 115.26 | 100.15 |
| 2 | 12.29 | 179.67 | 108.47 |
| 14 | 14.16 | 180.13 | 94.95 |
| 15 | 9.41 | 291.53 | 93.26 |
| 16 | 10.26 | 293.93 | 100.30 |
| 18 | 0.00 | 95.08 | 85.82 |
| 19 | 0.00 | 99.57 | 87.49 |
| 20 | 0.00 | 96.90 | 90.03 |
| 21 | 0.00 | 74.52 | 107.92 |
| 22 | 0.00 | 86.25 | 105.93 |
| 23 | 0.00 | 302.83 | 108.14 |
| 24 | 0.00 | 356.13 | 99.59 |

Example 1 and Example 2 were water-soluble matrix ointments and oil-based matrix ointments, respectively. Compared with Example 1 using water-soluble matrix, Example 2 using the oil-based ointment matrix showed a higher intradermal retention of active ingredients, which is beneficial for the active ingredients in local topical formulations to exert their pharmacological effects on the skin. Oil based ointment matrix can facilitate the retention of active substances in the skin.

The effect of viscosity regulators on the intradermal retention properties of active substances in ointment was studied in Examples 14, 15, and 16. Compared with Example 2, Example 14 showed comparable intradermal retention, while the combination of liquid paraffin and stearic acid did not improve the retention of active substances. Compared to Example 2, Examples 15 and 16 significantly increase the intradermal retention of active substances, which is beneficial for the active ingredients in topical formulations to exert their pharmacological effects on the skin.

The effect of solubilizers on the ability of active ingredients to remain in the skin in was studied in Examples 18, 19, and 20. The intradermal retention of the active substance in Examples 18, 19, and 20 was equivalent, but compared to Example 2, the intradermal retention was reduced. The addition of solubilizers was not conducive to the active ingredients in topical formulations exerting their pharmacological effects in the skin.

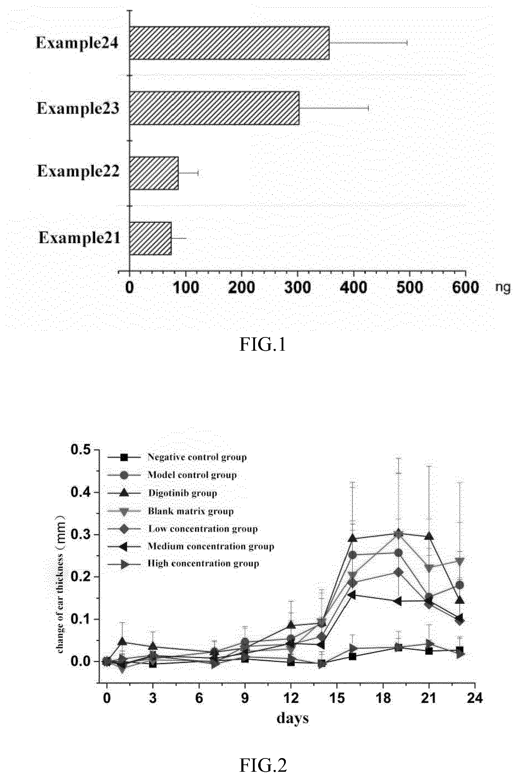

The relationship between the intradermal retention of active ingredients and the concentrations of active ingredients at 0.1%, 0.5%, 1%, and 2% was examined in Examples 21-24. With the increase of drug loading, the retention of active substances in pig skin showed an increasing trend, while the increase trend of high concentration delayed retention slowed down, as shown in FIG. 1.

### Experimental Example 2 Stability Study

The stability of the ointment prepared in Examples 21-24 was studied by placing it at 30 °C± 2 °C and RH 65% ± 5%, and the changes in appearance, content, and total impurities were examined. The results are shown in Table 13. The results indicate that Examples 21-24 exhibit good physical and chemical stability under accelerated conditions.

**Table 13**

| Example | Examining condition | Examining time (Month) | Property | Content (%) | Total impurities (%) |
|---|---|---|---|---|---|
| Example 21 | 30°C ± 2°C/ RH65% ± 5% | Start | Off-white ointment | 96.0 | 0.4% |
| | | 1M | Off-white ointment | 97.5 | 0.4% |
| Example 22 | 30°C ± 2°C/ RH65% ± 5% | Start | Off-white ointment | 99.7 | 0.4% |
| | | 1M | Off-white ointment | 100.2 | 0.4% |
| Example 23 | 30°C ± 2°C/ RH65% ± 5% | Start | Off-white ointment | 99.4 | 0.4% |
| | | 1M | Off-white ointment | 99.6 | 0.4% |
| Example 24 | 30°C ± 2°C/ RH65% ± 5% | Start | White ointment | 101.4 | 0.4% |
| | | 1M | White ointment | 101.7 | 0.4% |

### Experiment Example 3 Pharmacodynamic evaluation of DNCB induced atopic dermatitis model in rats using local topical preparations

### 1. Test purpose:

This study investigated the therapeutic effect of Examples 22-24 on a Brown North rat (BN rat) specific dermatitis model induced by applying 2,4-dinitrochlorobenzene (DNCB) in the ear.

### 2. Experimental animals:

The animal species was Brown Norway (BN) rats, aged 12-13 weeks, female, with a weight range of 120-200 g and a grade of SPF when transferred to the animal.

### 3. Main reagent preparation:

Acetone olive oil mixed solution (3:1): taking 10 mL as an example, 7.5 mL of acetone and 2.5 mL of olive oil were mixed well to obtain the solution.

0.5% DNCB solution: Taking the preparation of 10 mL as an example, 0.05 g of DNCB yellow crystals was weighed, added with 10 mL of acetone olive oil mixed solution (3:1), mixed well, and stored in the dark. It was prepared as needed, and can be prepared according to the proportion.

### 4. Dosage and group design of test and control substances (see Table 14):

**Table 14 List of Group and Dosage Design**

| Groups | Number of animals (no.) Female | Name of test animals or control substance | Dosage administered (µL/dose/ear) | Administration method |
|---|---|---|---|---|
| Negative control group | 5 (10 ears) | - | - | - |
| Model control group | 10 (10 ears) | - | - | - |
| Blank matrix group | 9 (9 ears) | Blank substance* | 20 | right ear transdermal administration |
| Positive control group | 9 (9 ears) | 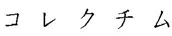 Ointment 0.5% | 20 | right ear transdermal administration |
| Low concentration group | 9 (9 ears) | Example 22 | 20 | right ear transdermal administration |
| Medium concentration group | 9 (9 ears) | Example 23 | 20 | right ear transdermal administration |
| High concentration group | 9 (9 ears) | Example 24 | 20 | right ear transdermal administration |

| | | | | |
|---|---|---|---|---|
| *Blank substance refers to the composition of all excipients except for the main components. | | | | |

### 5. Overview of experimental methods:

Modeling: Except for the negative control groups, all other animals were modeled. The modeling method was as follows: 40 µL 0.5% DNCB solution (acetone: olive oil=3:1) was applied evenly to the back and inner ear shells of the left and right ears of animals on D1, D3, D7, D9, D12, D14, D16, D19, D21, and D23. 40 µL acetone-olive oil mixed solution was applied evenly on the negative control group animals on the back and inner ear shells of the left and right ears. During the modeling period, animals were raised in a single box. After about 6 hours of modeling, the modeling area was gently clean with a cotton swab dipped in physiological saline.

Administration: animals in the positive control group, blank matrix group, low concentration group, medium concentration group, and high concentration group were administered to the right ear according to the settings. No treatment was performed on both ears of the negative control group and model control group, as well as on the left ear of the other experimental groups. During the administration period, the animals were raised in a single box after wearing Elizabeth collar, and after drug removal, they were raised in groups normally. On the day of modeling, the drug was administered approximately 1 hour after modeling, and medication was removed approximately 5 hours after administration. Starting from the day of the first modeling, the medication was administered once a day for 23 consecutive days.

Testing indicators: Ear thickness was measured approximately 6 hours after modeling at D0, D1, D3, D7, D9, D12, D14, D16, D19, D21, and D23. After the experiment, the animals were euthanized and the weight of approximately 8 mm ear pieces in the same area was measured. 6. Test results:

By comparing the changes in ear thickness and ear swelling, the therapeutic effect of Examples 22-24 on the Brown Normal rat (BN rat) atopic dermatitis model was investigated.

Ear thickness: according to the results of detecting changes in ear thickness indicators (as shown in Table 15 and FIG. 2), Examples 22-24 can suppress the increase in ear thickness with increasing concentration. The trend of ear thickness change in Example 22 is similar to that of the positive drug (0.5% ointment). Example 24 shows a significant advantage in inhibiting the increase in ear thickness.

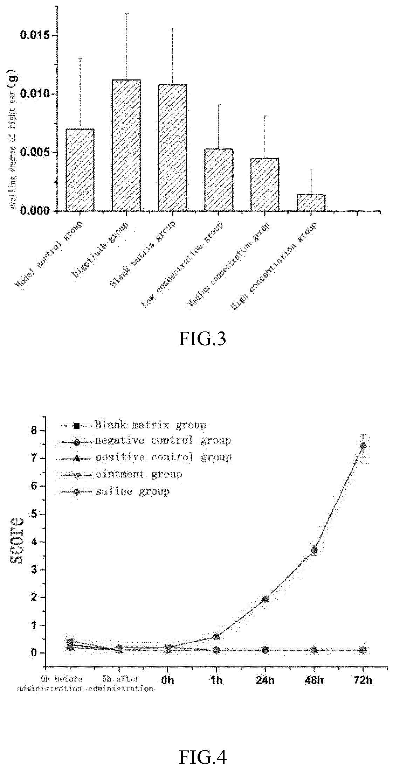

Ear swelling degree: after the experiment, the right ear swelling degree was calculated by measuring the weight of approximately 8mm ear pieces in the same area (ear swelling degree=measurement group ear piece weight-negative control group ear piece weight). The results are shown in Table 16 and FIG. 3. The swelling degree of the right ear is consistent with the change in ear thickness. The ointment preparation has shown an advantage in inhibiting ear swelling from Example 23, while Example 24 has shown a significant advantage in inhibiting ear swelling.

**Table 15 Statistical Table of Changes in Right Ear Thickness in Rats**

| Time | Right ear swelling degree (mm) | | | | | | |
|---|---|---|---|---|---|---|---|
| | Negativ e control | Model contro l | Digotini b group | Blank group | Low concentratio n group | Medium concentratio n group | High concentratio n group |
| Before administratio n | 0.000 ± 0.000 | 0.000 ± 0.000 | 0.000 ± 0.000 | 0.000 ± 0.000 | 0.000 ± 0.000 | 0.000 ± 0.000 | 0.000 ± 0.000 |
| 1 | 0.000 ± 0.025 | -0.004 ± 0.020 | 0.046 ± 0.046 | -0.01 6 ± 0.02 | -0.008 ± 0.021 | -0.006 ± 0.024 | 0.006 ± 0.033 |
| 3 | -0.006 ± 0.031 | 0.007 ± 0.027 | 0.035 ± 0.035 | 0.004 ± 0.021 | 0.012 ± 0.011 | 0.014 ± 0.024 | 0.015 ± 0.027 |
| 7 | 0.002 ± 0.030 | 0.023 ± 0.024 | 0.022 ± 0.027 | 0.001 ± 0.030 | -0.002 ± 0.014 | 0.008 ± 0.012 | -0.006 ± 0.018 |
| 9 | 0.006 ± 0.020 | 0.046 ± 0.037 | 0.032 ± 0.024 | 0.024 ± 0.026 | 0.036 ± 0.043 | 0.019 ± 0.022 | 0.011 ± 0.018 |
| 12 | -0.002 ± 0.019 | 0.054 ± 0.043 | 0.085 ± 0.058 | 0.030 ± 0.051 | 0.041 ± 0.052 | 0.043 ± 0.072 | 0.007 ± 0.011 |
| 14 | -0.004 ± 0.027 | 0.090 ± 0.080 | 0.091 ± 0.058 | 0.095 ± 0.067 | 0.059 ± 0.046 | 0.040 ± 0.060 | -0.006 ± 0.023 |
| 16 | 0.012 ± 0.016 | 0.252 ± 0.160 | 0.290 ± 0.133 | 0.205 ± 0.128 | 0.186 ± 0.124 | 0.158 ± 0.167 | 0.031 ± 0.032 |
| 19 | 0.033 ± 0.022 | 0.257 ± 0.187 | 0.303 ± 0.177 | 0.301 ± 0.144 | 0.211 ± 0.126 | 0.143 ± 0.160 | 0.034 ± 0.037 |
| 21 | 0.025 ± 0.026 | 0.153 ± 0.114 | 0.295 ± 0.166 | 0.222 ± 0.115 | 0.136 ± 0.115 | 0.144 ± 0.149 | 0.042 ± 0.045 |
| 23 | 0.027 ± 0.031 | 0.181 ± 0.148 | 0.144 ± 0.116 | 0.238 ± 0.184 | 0.097 ± 0.101 | 0.103 ± 0.091 | 0.018 ± 0.036 |

| | | | | | | | |
|---|---|---|---|---|---|---|---|
| Note: Ear swelling degree (mm)=measured ear thickness on the day of measurement-ear thickness before administration. | | | | | | | |

**Table 16 Ear Weight Statistics**

| Groups | Left ear weight (g) | Right ear weight (g) | Right ear swelling degree (g) |
|---|---|---|---|
| Negative control group | 0.0271±0.0025 | | - |
| Model control group | 0.0335±0.0051## | 0.0340±0.0060## | 0.0070±0.0060## |
| Digotinib group | 0.0378±0.0047 | 0.0382±0.0057 | 0.0112±0.0057 |
| Blank matrix group | 0.0349±0.0038## | 0.0379±0.0048## | 0.0108±0.0048## |
| Low concentration group | 0.0307±0.0033A | 0.0324±0.0038A | 0.0053±0.0038A |
| Medium concentration group | 0.0305±0.0038A | 0.0315±0.0037▲▲ | 0.0045±0.0037▲▲ |
| High concentration group | 0.0271±0.0022**▲▲ | 0.0285±0.0022*▲▲ | 0.0014±0.0022*▲▲ |

| | | | |
|---|---|---|---|
| Note: Ear swelling=weight of the measured group's ear piece minus weight of the negative control group's ear piece. Animals in the administration group were not given medication in the left ear, but given medication in the right ear. Compared with the negative control group, the model control group and blank matrix group had # P<0.05, # # P<0.01. Compared with the model control group, the positive control group, blank matrix group, and administration group * showed P<0.05, * * P<0.01. Compared with the blank matrix group, the administration group showed 1 P<0.05 and ▲▲ P<0.01. | | | |

### Experimental Example 4 Local Irritation Test of Formulation

1. intact skin irritation test: about 24 hours before the test, a pet trimmer was used to cut off the long hair on both sides of the rabbit's back spine, and then an electric shaver was used to remove the short hair without damaging the epidermis. The experiment adopted a self-control method, in which each white rabbit was processed to obtain four administration areas, with a hair removal range of about 3 cm in each area × 3 cm, administration area of 2.5 cm × 2.5 cm. For the ointment preparation group, approximately 0.5 g of homemade ointment was applied directly to the surface of the skin in Example 24. For the positive control group, approximately 0.5 mL of 10% sodium dodecyl sulfate was applied to the surface of the skin. For the blank matrix group, approximately 0.5 g of blank matrix was applied to the skin surface. For the physiological saline group, approximately 0.5 mL of physiological saline was applied to the surface of the skin. Another area was used as the negative control group. Afterwards, they were covered by double-layer gauze and a layer of glass paper (2.5 cm) × with 2.5 cm and secured with medical anti-allergic tape. A closed test was performed, residual test substance on the skin surface was wiped off with warm water after application. The skin reaction at the application site was observed at 0 h, 1 h, 24 h, 48 h, and 72 h after removing the test substance, and evaluated regarding the skin reaction score according to Table 17. The average score of the test animal skin evaluation was used for comprehensive evaluation, and the skin irritation intensity was determined according to Table 18.

**Table 17 Scoring criteria for skin irritation reactions**

| Stimulus response | Score |
|---|---|
| Erythema | |
| No erythema | 0 |
| Mild erythema (barely visible) | 1 |
| Moderate erythema (clearly visible) | 2 |
| Severe erythema | 3 |
| Purple red erythema to mild scab formation | 4 |
| Edema | |
| No edema | 0 |
| Mild edema (barely visible) | 1 |
| Moderate edema (obvious protrusion) | 2 |
| Severe edema (skin protrusion of 1mm, clear contour) | 3 |
| Severe edema (skin protrusion of more than 1mm with | 4 |
| The highest total score | 8 |

**Table 18 Evaluation criteria for skin irritation intensity**

| Score | Evaluation |
|---|---|
| 0∼0.49 | No irritation |
| 0.5∼2.99 | Mild irritation |
| 3.0∼5.99 | Moderate irritation |
| 6.0∼8.00 | severe irritation |

The results are shown in Table 19 and FIG. 4. Based on the mean score of individual observation points, the skin irritation intensity levels of individual groups were evaluated by comparing Table 17 and Table 18. The positive control group showed severe irritation, while the ointment preparation group, the physiological saline group, and the blank matrix group in Example 24 showed no irritation.

**Table 19 Scoring table for complete skin irritation test**

| Group | Before administrati on | | 5h after administrati on | | 0h after removing preparatio n | | 1h after removing preparatio n | | 24h after removing preparatio n | | 48h after removing preparation | | 72h after removing preparation | |
|---|---|---|---|---|---|---|---|---|---|---|---|---|---|---|
| | Sc. | Ev. | Sc. | Ev. | Sc. | Ev. | Sc. | Ev. | Sc. | Ev. | Sc. | Ev. | Sc. | Ev. |
| Ointme nt group | 0.43 | No | 0.20 | No | 0.20 | No | 0.1 0 | No | 0.1 0 | No | 0.1 0 | No | 0.1 0 | No |
| Blank matrix group | 0.30 | No | 0.18 | No | 0.20 | No | 0.1 0 | No | 0.1 0 | No | 0.1 0 | No | 0.1 0 | No |
| Negativ e control group | 0.20 | No | 0.20 | No | 0.20 | No | 0.2 0 | No | 0.1 0 | No | 0.1 0 | No | 0.1 0 | No |
| Saline group | 0.20 | No | 0.20 | No | 0.20 | No | 0.2 0 | No | 0.1 0 | No | 0.1 0 | No | 0.1 0 | No |
| Positive control group | 0.20 | No | 0.20 | No | 0.20 | No | 0.5 8 | Mil d | 1.9 3 | Mil d | 3.7 0 | Modera te | 7.4 5 | Sever e |

2. **Single dose irritation test on damaged skin:** about 24 hours before the test, a pet trimmer was used to cut off the long hair on both sides of the rabbit's back spine, then an electric shaver was used to remove the short hair, and manual treatment was performed to cause skin damage. The experiment was performed by using a self-control method, in which each white rabbit was processed to obtain two areas, with a hair removal range of about 3 cm × 3 cm in each area, and administration area of 2.5 cm × 2.5 cm. For the administration group, approximately 0.5 g of Example 24 was applied directly to the surface of the damaged skin. Another area was used as a blank control. Afterwards, they were covered by using a double-layer gauze and a layer of glass paper (2.5 cm) × with 2.5 cm and secured with medical anti allergic tape. A closed trial was adopted, administer was performed for 5 hours, and after application, residual test substance on the skin surface was wiped off with warm water. The skin irritation response of the application site was observed at 0 h, 1 h, 24 h, 48 h, and 72 h after removing the test substance. A skin reaction score was performed according to Table 17, and comprehensively evaluated regarding the average score of the test animal skin evaluation. The skin irritation intensity was determined according to Table 18.

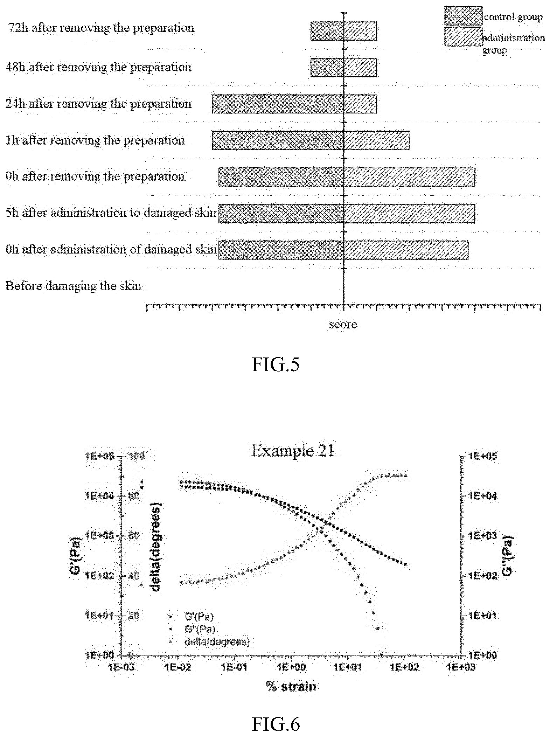

As shown in Table 20 and FIG. 5, damaged skin administration group (Example 24) and damaged skin control group (skin damaged but no administration) had the same irritating reaction process of erythema and edema. Example 24 did not exacerbate the erythema and edema of the damaged skin, indicating that the ointment was not irritation to the damaged skin.

**Table 20 Score of single administration irritation test for damaged skin**

| Time points | Score | | Evaluation | |
|---|---|---|---|---|
| | Administration group | Blank group | Administration group | Blank group |
| Before skin damage | 0.0 | 0.0 | No | No |
| 0h before administration after skin damage | 3.8 | 3.8 | Moderate | Moderate |
| 5h after administration after skin damage | 4.0 | 3.8 | Moderate | Moderate |
| 0h after removing preparation from damaged skin | 4.0 | 3.8 | Moderate | Moderate |
| 1h after removing preparation from damaged skin | 2.0 | 4.0 | Mild | Moderate |
| 24h after removing preparation from damaged skin | 1.0 | 4.0 | Mild | Moderate |
| 48h after removing preparation from damaged skin | 1.0 | 2.0 | Mild | Mild |
| 72h after removing preparation from damaged skin | 1.0 | 1.0 | Mild | Mild |

### Experiment Example 5 Rheology

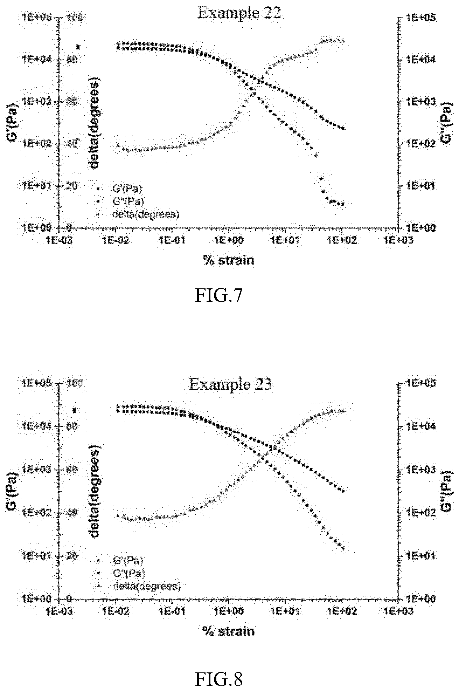

By using strain scanning in vibration mode, the flow point where the elastic modulus G 'intersects with the viscous modulus G' was observed. The corresponding force was the force that can cause the paste to flow. The extensibility of the ointment applied to the skin was examined. The lower the flow point, the better the skin extensibility. The extensibility of Example 21 ointment was the same as that of the blank matrix, which was 0.15 (% strain). There was no significant change in the extensibility of the ointments in Examples 22-24, all of which were around 0.4 (% train). There was no significant difference. The results are shown in Table 21 and Figures 6, 7, 8, and 9.

**Table 21 Elastic modulus G ', viscous modulus G' 'and phase angle within the linear viscoelastic zone range δ average value**

| Linear viscoelastic zone parameter | G'average (Pa) | G" average (Pa) | δ (degrees) |
|---|---|---|---|
| Example 21 | 22158 | 16707 | 37.0 |
| Example 22 | 23198 | 18076 | 37.9 |
| Example 23 | 28092 | 21820 | 37.8 |
| Example 24 | 22725 | 18553 | 37.0 |

### Experimental Example 6 Determination of Single Dosage Curve in Bama Small Pigs

1. **Experiment purpose:** to investigate the pharmacokinetic characteristics of compound II prepared by single percutaneous application or intravenous injection in Example 24 of Bama miniature pigs, and to calculate relevant pharmacokinetic parameters.
2. **Experiment animals:** two 3-4 months old ordinary grade Bama miniature pigs (half male and half female), weighing approximately 10-15 kg, with individual weight values within the mean ± 20% range.
3. Solution preparation:
Solvent: 5% DMSO (dimethyl sulfoxide)/95% (20% hydroxypropyl)-β-Cyclodextrin aqueous solution;
Preparation method: weighing an appropriate amount of compound II, adding a certain amount of DMSO, and then slowly adding the corresponding volume of 20% hydroxypropyl group- β- Cyclodextrin, vortex and sonicate to obtain a clear solution, the solution is filtrated via 0.22 µ M filter membrane.

4. Group design:
The groups were divided into intravenous injection group and percutaneous application group. Two groups shared two animals and were reused after each cycle of elution. Using the PRISTIMA 7.2.0 data system, Bama miniature pigs were randomly divided by gender based on their body weight.
5. Dose design:
The intravenous injection group in this experiment received a dosage of 0.2 mg/kg (concentration of 0.1 mg/mL), while the transdermal application group used concentration of the ointment prepared in Example 24 as the application concentration. A thickness of 0.03 g/cm² was applied transdermally, with a dosage of 0.6 mg/cm² (based on active ingredients) and an application area of approximately 5% of the body surface area of Bama miniature pigs. Bama miniature pigs were first given compound II by intravenous injection, and after 7 days of elution, the ointment prepared in Example 24 was applied transdermally.
6. Administration:
6.1 Intravenous injection group
   Ear vein injection, single dose (the dosage for each animal was adjusted based on the most recent measured body weight).
6.2 Transdermal application group
   Transdermal application was performed on the back skin, single dosage.
   Preparation before administration: before administration, an electric shaver was used to remove the fur on the back of small pigs for skin preparation. The skin preparation area was greater than 5% of the body surface area of each small pig, and marked with a marker pen before administration, for which attention was paid to observe the skin condition before administration to avoid damage or redness;
   Formula for calculating body surface area: body surface area (cm²) =9.0×Weight (g) ^{2/3};
   Administration: the required amount of the test substance (Example 24) was accurately weighed and applied evenly to the skin preparation area on the back of the small pig. The administration amount and area of each animal were calculated based on the most recent weight measurement. The total amount of ointment administration was equal to the administration area multiplied by the administration amount (calculated as ointment).

7. Sample collection and processing
7.1 Collection time
   **Sampling time for intravenous injection group:** 5 minutes (± 1 minute), 15 minutes (± 1 minute), 30 minutes (± 1 minute), 1 hour (± 2 minutes), 2 hours (± 2 minutes), 4 hours (± 5 minutes), 6 hours (± 5 minutes), 8 hours (± 5 minutes), 12 hours (± 10 minutes), 24 hours (± 10 minutes), and 48 hours (± 10 minutes) before and after administration.
   **Sampling time for percutaneous application group:** 10 minutes (± 1 minute), 30 minutes (± 1 minute), 1 hour (± 2 minutes), 2 hours (± 2 minutes), 4 hours (± 5 minutes), 6 hours (± 5 minutes), 8 hours (± 5 minutes), 12 hours (± 10 minutes), 24 hours (± 10 minutes), 36 hours (± 10 minutes), and 48 hours (± 10 minutes) before and after administration.
7.2 Sampling method and blood sample processing:
   All surviving Bama miniature pigs in each group were sampled from the jugular vein, with a sampling volume of approximately 1.0 mL, in which anticoagulant EDTA-K2 was used.
   The whole blood sample was placed in an ice box before centrifugation, maintained at 2-8 °C, and centrifuged at 1800g for 10 minutes. Plasma was separated, and stored below -66 °C.

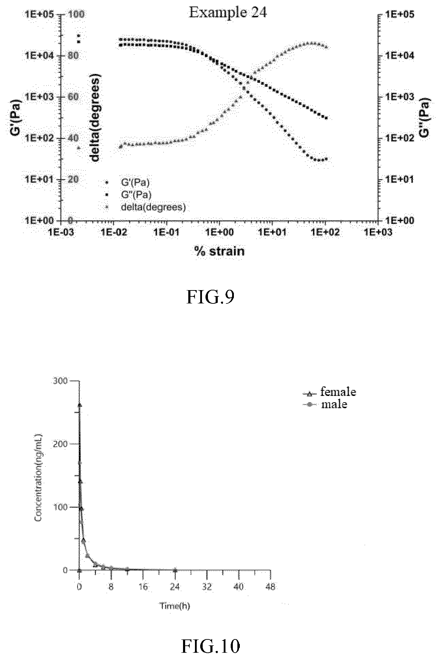

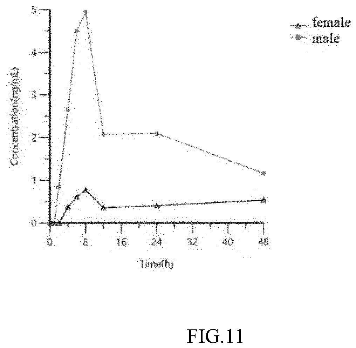

8. Results
After intravenous and percutaneous administration, the plasma drug concentrations of compound II in male and female Bama miniature pigs are shown in Tables 22 and 23, the plasma pharmacokinetic parameters are shown in Tables 24 and 25, and the plasma drug concentration curves are shown in FIGs. 10 and 11.
After a single intravenous administration of 0.2 mg/kg of compound II to both male and female Bama miniature pigs, the elimination half-life (T_{1/2}) of compound II and the area under the time plasma concentration curve (AUC₀₋ₗₐₛₜ) from 0 to the last quantifiable time point were 4.71 h and 225 h • ng/mL, respectively.
After a single percutaneous application of the ointment prepared in Example 24, the average peak time (Tₘₐₓ), peak concentration (Cₘₐₓ), and AUC₀₋ₗₐₛₜ values of compound II for both males and females were 12 h, 2.86 ng/mL, and 70 h • ng/mL, respectively.
After a single percutaneous application of the ointment prepared in Example 24 on male and female Bama miniature pigs, the bioavailability of compound II was 0.60%.

9. Conclusion
The ointment prepared in Example 24 had little absorption into the bloodstream after transdermal administration, and its bioavailability is very low. It effectively reduces the risk of systemic immune suppression of Compound II and increases the local skin action concentration of Compound II, achieving the goal of reducing toxicity and increasing efficacy.

**Table 22: Plasma drug concentration after single intravenous injection of 0.2 mg/kg compound II in male and female Bama miniature pigs (ng/mL)**

| Time (h) | Female | Male |
|---|---|---|
| 0 | 0 | 0 |
| 0.08333 | 262 | 170 |
| 0.25 | 141 | 104 |
| 0.5 | 98 | 75.6 |
| 1 | 48.4 | 44.3 |
| 2 | 23.3 | 23.6 |
| 4 | 8.62 | 10.7 |
| 6 | 4.94 | 6.25 |
| 8 | 3.02 | 4.01 |
| 12 | 1.59 | 2.23 |
| 24 | 0.286 | 0.375 |
| 48 | 0 | 0 |

**Table 23: Plasma drug concentration after single percutaneous application of ointment prepared in Example 24 for male and female Bama miniature pigs (ng/mL)**

| Time (h) | Female | Male |
|---|---|---|
| 0 | 0 | 0 |
| 0.16667 | 0 | 0 |
| 0.5 | 0 | 0 |
| 1 | 0 | 0 |
| 2 | 0 | 0 |
| 4 | 0 | 0 |
| 6 | 0.375 | 2.65 |
| 8 | 0.612 | 4.49 |
| 12 | 0.771 | 4.94 |
| 24 | 0.362 | 2.08 |
| 36 | 0.411 | 2.1 |
| 48 | 0.544 | 1.17 |

**Table 24: Pharmacokinetic parameters after a single intravenous injection of 0.2 mg/kg compound II in male and female Bama miniature pigs**

| Group | t1/2 | Tmax | Cmax | AUC 0-last |
|---|---|---|---|---|
| | (h) | (h) | (ng/mL) | (h* ng/mL) |
| Female | 4.74 | 0.083 | 262 | 236 |
| Male | 4.68 | 0.083 | 170 | 215 |
| Average | 4.71 | 0.083 | 216 | 225 |

**Table 25 Pharmacokinetic parameters after single percutaneous application of ointment prepared in Example 24 for male and female Bama miniature pigs**

| Group | t1/2 | Tmax | Cmax | AUC 0-last |
|---|---|---|---|---|
| | (h) | (h) | (ng/mL) | (h* ng/mL) |
| Female | - | 12 | 4.94 | 118 |
| Male | - | 12 | 0.771 | 22 |
| Average | - | 12 | 2.86 | 70 |

| | | | | |
|---|---|---|---|---|
| Note: "-" indicates that Rseq (Adjusted)<0.7, fitting not calculated λ, in which the relevant parameters have not been calculated. | | | | |

## Claims

1. An local topical preparation containing a JAK inhibitor, **characterized in that**, the local topical preparation comprises the JAK inhibitor, a matrix and an additive agent, wherein the JAK inhibitor comprises a compound of formula (I), an isomer thereof, pharmaceutically acceptable salts thereof, or crystal form A thereof 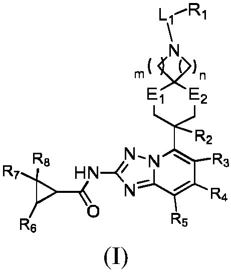 where,
E₁ and E₂ are independently selected from a group consisting of single bond, -CH₂- or -(CH₂)₂-, respectively;
L₁ is selected from single bond, -(CH₂)_{g}-, -c (=O)- or -c(=O)-(CH₂)ₕ-;
m is 1 or 2;
n is 1 or 2;
g is 1, 2 or 3;
h is 1, 2 or 3;
R₁ is selected from H, CN, C₁₋₆ alkyl or 3-6-membered cycloalkyl, wherein the C₁₋₆ alkyl and 3-6-membered cycloalkyl are optionally substituted by 1, 2 or 3 Rₐ;
R₂ is selected from H, F, Cl, Br, I or C₁₋₃ alkyl, wherein the C₁₋₃ alkyl is optionally substituted by 1, 2 or 3 R_{b};
R₃, R₄ and R₅ are independently selected from H, F, Cl, Br, I or C₁₋₃ alkyl, respectively, wherein the C₁₋₃ alkyl is optionally substituted by 1, 2 or 3 R_{c};
R₆, R₇ and R₈ are independently selected from H, F, Cl, Br, I or C₁₋₃ alkyl, respectively, wherein the C₁₋₃ alkyl is optionally substituted by 1, 2 or 3 R_{d};
Each Rₐ is independently selected from H, F, Cl, Br, I, CN or C₁₋₃ alkyl, respectively, wherein the C₁₋₃ alkyl is optionally substituted by 1, 2 or 3 R;
Each R_{b} is independently selected from F, Cl, Br or I;
Each R_{c} is independently selected from F, Cl, Br or I;
Each R_{d} is independently selected from F, Cl, Br or I; and
Each R is independently selected from F, Cl, Br or I.

2. The local topical preparation according to claim 1, **characterized in that**, in the compound of formula (I), isomers thereof, or pharmaceutically acceptable salts thereof, each Rₐ is independently selected from H, F, Cl, Br, I, or CN.

3. The local topical preparation according to claim 1 or 2, **characterized in that**, in the compound of formula (I), isomers thereof, or pharmaceutically acceptable salts thereof, R₁ is selected from H, CN, C₁₋₃ alkyl or 3-5-membered cycloalkyl, wherein the C₁₋₃ alkyl and 3-5-membered cycloalkyl are optionally substituted by 1, 2, or 3 Ra.

4. The local topical preparation according to claim 3, **characterized in that**, in the compound of formula (I), isomers thereof, or pharmaceutically acceptable salts thereof, R₁ is selected from H, CN, CH₃, 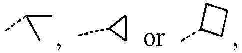 wherein CH₃, 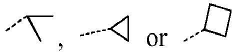 is optionally substituted by 1, 2, or 3 Ra.

5. The local topical preparation according to claim 4, **characterized in that**, in the compound of formula (I), isomers thereof, or pharmaceutically acceptable salts thereof, R₁ is selected from H, CN, CF₃, CHF₂,

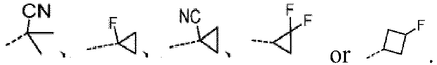

6. The local topical preparation according to claim 1, **characterized in that**, in the compound of formula (I), isomers thereof, or pharmaceutically acceptable salts thereof, R₂ is selected from H, F, Cl, Br, or I.

7. The local topical preparation according to claim 1, **characterized in that**, in the compound of formula (I), isomers thereof, or pharmaceutically acceptable salts thereof, R₃, R₄, and R₅ are independently selected from H, F, Cl, Br, or I.

8. The local topical preparation according to claim 1, **characterized in that**, in the compound of formula (I), isomers thereof, or pharmaceutically acceptable salts thereof, R₆, R₇, and R₈ are independently selected from H, F, Cl, Br, or I.

9. The local topical preparation according to claim 1, **characterized in that**, in the compound of formula (I), isomers thereof, or pharmaceutically acceptable salts thereof, L₁ is selected from a single bond, -CH₂-, -(CH₂)₂-, -C(=O)- or -C(=O)-(CH₂)-.

10. The local topical preparation according to claim 1, **characterized in that**, in the compound of formula (I), isomers thereof, or pharmaceutically acceptable salts thereof, the structural unit 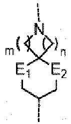 is selected from

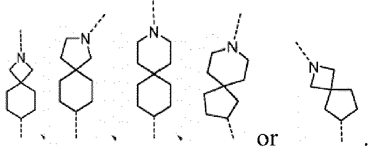

11. The local topical preparation according to claim 1, **characterized in that**, in the compound of formula (I), isomers thereof, or pharmaceutically acceptable salts thereof, the structural unit 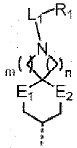 is selected from

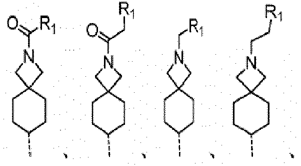

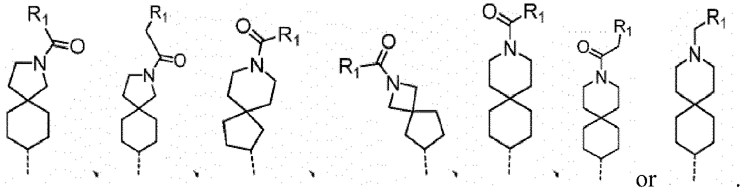

12. The local topical preparation according to claim 1, **characterized in that**, in the compound of formula (I), isomers thereof, or pharmaceutically acceptable salts thereof, the structural unit 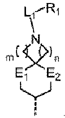 is selected from

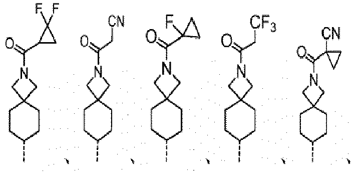

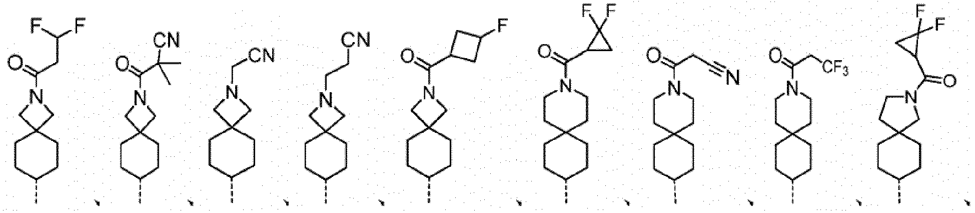

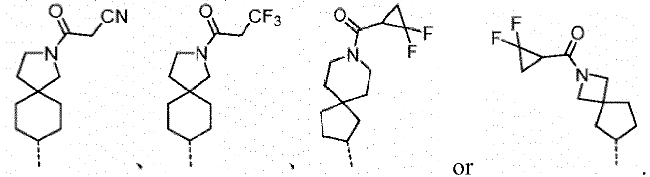

13. The local topical preparation according to any one of claims 1-9, **characterized in that**, the compound of formula (I), isomers thereof, or pharmaceutically acceptable salts thereof is selected from 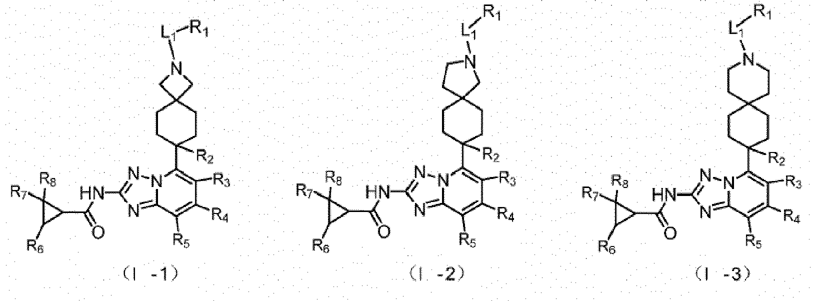 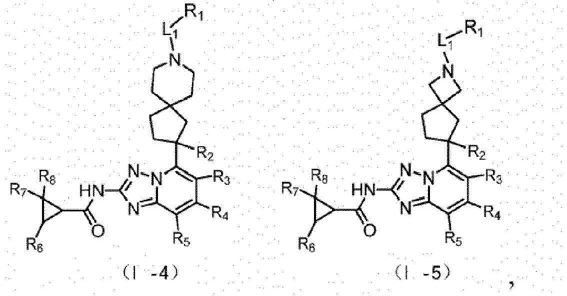 where
L₁ is defined in claim 1 or 9;
R₁ is defined in claims 1-5;
R₂ is defined in claim 1 or 6;
R₃, R₄, and R₅ is defined in claim 1 or 7; and
R₆, R₇, and R₈ is defined in claim 1 or 8.

14. The local topical preparation according to claim 13, **characterized in that**, the compound of formula (I), isomers thereof, or pharmaceutically acceptable salts thereof is selected from 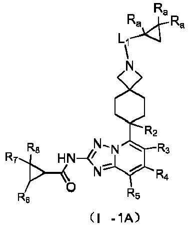 where,
L₁ is defined in claim 1 or 9;
Rₐ is defined in claim 1 or 2;
R₂ is defined in claim 1 or 6;
R₃, R₄, and R₅ are defined in claim 1 or 7;
R₆, R₇, and R₈ are defined in claim 1 or 8.

15. An local topical preparation containing a JAK inhibitor, **characterized in that**, the JAK inhibitor comprises the following compounds, their isomers, or pharmaceutically acceptable salts thereof

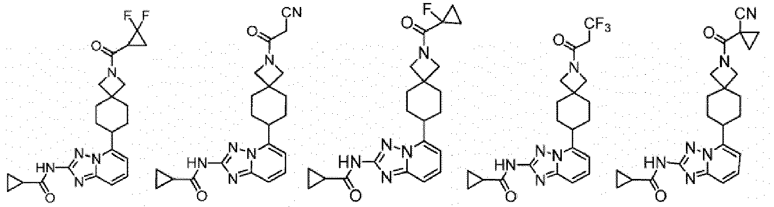

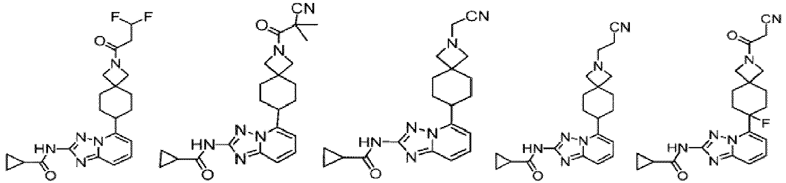

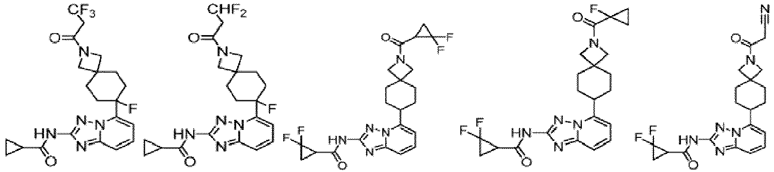

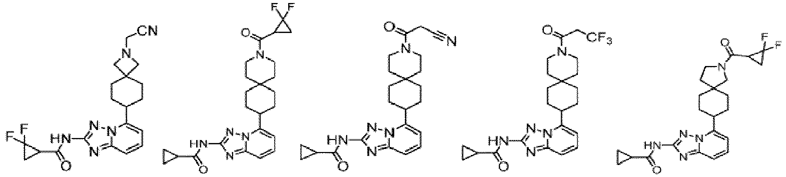

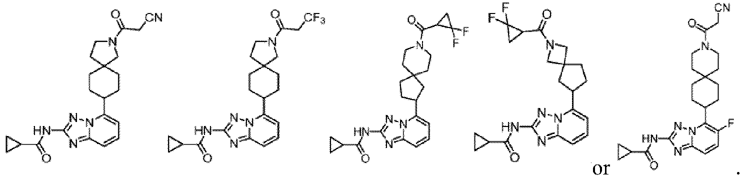

16. The local topical preparation according to claim 15, **characterized in that**, the compound of formula (I), isomers thereof, or pharmaceutically acceptable salts thereof is selected from

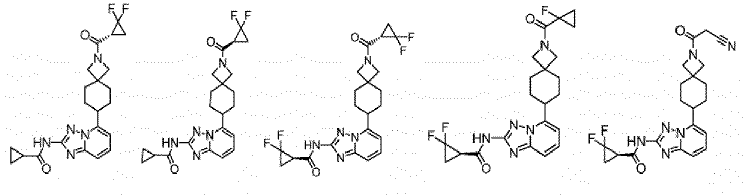

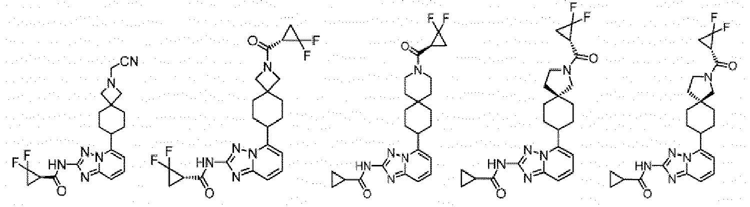

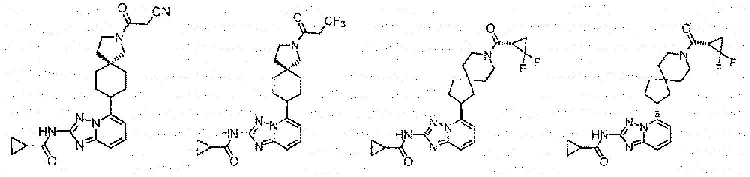

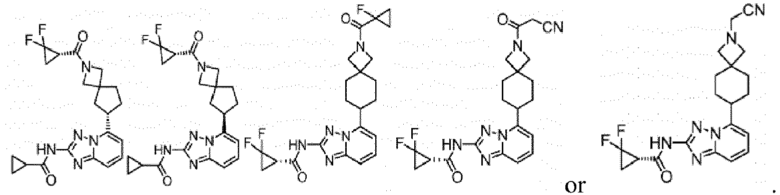

17. The local topical preparation according to claims 1-16, **characterized in that**, the matrix is selected from an oil soluble matrix, a water-soluble matrix, or a combination of two or more of them.

18. The topical formulation according to claim 17, **characterized in that**, the oil soluble matrix is selected from oil substances, hydrocarbons, lipids, synthetic (semi synthetic) oil substances, or a combination of two or more of them.

19. The topical preparation according to claim 18, **characterized in that**, the oil substances are selected from porcine fat, sheep fat, cow fat, sesame oil, sesame oil, cottonseed oil, soybean oil, peanut oil, olive oil, vegetable oil, hydrogenated vegetable oil, or a combination of two or more of them.

20. The local topical preparation according to claim 18, **characterized in that**, the hydrocarbons are selected from white vaseline, yellow vaseline, liquid paraffin, white wax, paraffin, microcrystalline paraffin, ground wax, Sasol paraffin, or a combination of two or more of them.

21. The topical preparation according to claim 18, **characterized in that**, the lipids are selected from lanolin, beeswax, whale wax, or a combination of two or more of them.

22. The local topical preparation according to claim 18, **characterized in that**, the hydrocarbons are selected from white vaseline, yellow vaseline, liquid paraffin, white wax, paraffin, microcrystalline paraffin, ground wax, Sasol paraffin, or a combination of two or more of them.

23. The topical preparation according to claim 18, **characterized in that**, the oil substances are selected from lanolin, beeswax, whale wax, or a combination of two or more of them.

24. The topical preparation according to claim 18, **characterized in that**, the synthesized (semi synthetic) oil substances are selected from squalane, lanolin derivatives, lanolin alcohol, acetylated lanolin, polyoxyethylene lanolin, hydrogenated lanolin, silicone, glyceryl behenate, palmitic acid, stearic acid, isostearic acid, octadecanol, cetanol, stearanol, or a combination of two or more of them.

25. The topical preparation according to claim 17, **characterized in that**, the water-soluble matrix is selected from polyethylene glycol 3350 (PEG 3350), polyethylene glycol 400 (PEG 400), cellulose derivatives, or two or more combinations thereof.

26. The local topical preparation according to claims 1-16, **characterized in that**, the additive comprises one or more antioxidants.

27. The topical preparation according to claim 26, **characterized in that**, the antioxidant is selected from sodium bisulfite, sodium metabisulfite, sodium thiosulfate, sodium sulfite, vitamin C, cysteine, methionine, tert butyl p-hydroxyanisole, dibutylhydroxytoluene, propyl gallate, vitamin E, edetic acid (EDTA), citric acid, tartaric acid, or a combination of two or more of them.

28. The local topical preparation according to claims 1-16, **characterized in that**, the additive comprises one or more preservatives.

29. The topical preparation according to claim 28, **characterized in that**, the preservative is selected from trichloro tert butanol, benzoic acid, sorbic acid, phenol, cresol, hydroxybenzyl methyl ester, hydroxybenzyl ethyl ester, benzalkonium bromide, benzalkonium chloride, or a combination of two or more of them.

30. The local topical preparation according to claims 1-16, **characterized in that**, the additive comprises one or more moisturizing agents.

31. The topical preparation according to claim 30, **characterized in that**, the moisturizing agent is selected from glycerol, propylene glycol, sorbitol, or a combination of two or more of them.

32. The local topical preparation according to claims 1-16, **characterized in that**, the additive comprises one or more solubilizers.

33. The topical preparation according to claim 32, **characterized in that**, the solubilizer is selected from monoglyceryl linoleic acid, diethylene glycol monoethyl ether, polyglycerol fatty acid ester, or a combination of two or more of them.

34. The local topical preparation according to claims 1-33, **characterized in that**, the local topical preparation comprises the JAK inhibitor, one or more matrices, one or more additive agents, wherein the JAK inhibitor is a compound of structural formula (II), formula (III), formula (IV), formula (V), or formula (VI) and its isomer, or a pharmaceutically acceptable salt thereof, or one of its crystal forms:

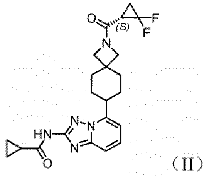

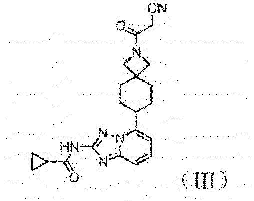

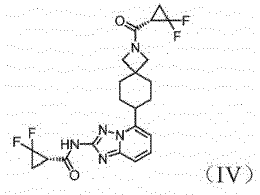

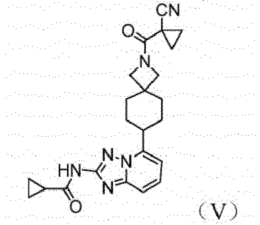

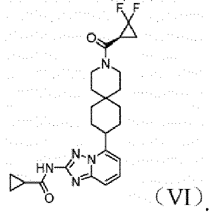

35. The local topical preparation according to claim 34, **characterized in that**, a content of JAK inhibitors is 0.01% to 10%, preferably 0.02% to 9%, further preferably 0.03% to 8%, further preferably 0.04% to 7%, most preferably 0.05%, 0.06%, 0.07%, 0.08%, 0.09%, 0.1%, 0.125%, 0.2%, 0.25%, 0.3%, 0.4%, 0.5%, 0.6%, 0.7%, 0.8%, 0.9%, 1%, 1.1%, 1.2%, 1.25%, 1.3%, 1.4%, 1.5%, 1.6% 1.7%, 1.8%, 1.9%, 2%, 2.25%, 2.5%, 2.8%, 3%, 3.2%, 3.5%, 3.6%, 3.8%, 4%, 4.5%, 5%, 5.5%, 6%, or 6.5% of a total weight of the local topical preparation.

36. The topical preparation according to claim 34, **characterized in that**, one or more matrices are selected from an oil soluble matrix.

37. The local topical preparation according to claim 36, **characterized in that**, the oil soluble matrix is selected from white vaseline, white wax, liquid paraffin, paraffin, Sasol paraffin, microcrystalline paraffin, or a combination of two or more of them.

38. The topical preparation according to claim 37, **characterized in that**, a content of white vaseline is 45% to 96%, preferably 50% to 95%, more preferably 53% to 94%, further preferably 55% to 93%, further preferably 56% to 92%, most preferably 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 80.5%, 81%, 81.5% 82%, 82.5%, 83%, 83.5%, 84%, 84.5%, 85%, 85.5%, 86%, 86.5%, 87%, 87.5%, 88%, 88.5%, 89%, 89.5%, 90%, 90.25%, 90.3%, 90.35%, 90.4%, 90.45%, 90.46%, 90.48%, 90.5%, 90.6%, 90.7%, 90.8%, 90.85%, 90.9%, 91%, 91.25%, 91.45%, or 91.5% of the total weight of the topical preparation.

39. The local topical preparation according to claim 37, **characterized in that**, a content of white wax is 0.5% to 10%, preferably 1% to 9%, more preferably 2% to 8%, further preferably 3% to 6%, more preferably 4% to 5%, most preferably 4.01%, 4.02%, 4.04%, 4.06%, 4.08%, 4.1%, 4.12%, 4.14%, 4.14%, 4.16%, 4.18%, 4.2%, 4.22%, 4.24%, 4.26%, 4.28%, 4.3%, 4.32%, 4.34%, 4.36%, 4.38%, 4.4%, 4.45%, 4.5% 4.55%, 4.6%, 4.65%, 4.7%, 4.75%, 4.8%, 4.85%, 4.9%, or 4.95% of the total weight of the local topical preparation.

40. The local topical preparation according to claim 37, **characterized in that**, a content of liquid paraffin is 0.2% to 50%, preferably 1% to 40%, more preferably 1.5% to 30%, further preferably 2% to 20%, more preferably 2.5% to 15%, most preferably 3%, 3.5%, 4%, 4.05%, 4.1%, 4.15%, 4.2%, 4.25%, 4.3%, 4.35%, 4.4%, 4.45%, 4.5%, 5%, 5.02%, 5.04%, 5.05%, 5.1%, 5.15%, 5.2%, 5.25%, 5.3% 5.35%, 5.4%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, or 14.5%. of the total weight of the local topical preparation

41. The topical preparation according to claim 37, **characterized in that**, a content of paraffin is 1% to 8%, preferably 2% to 7%, most preferably 3%, 3.5%, 4%, 4.5%, 5%, 5.5%, 6%, or 6.5% of the total weight of the topical preparation.

42. The local topical preparation according to claim 37, **characterized in that**, a content of Sasol paraffin is 1% to 10%, preferably 1.5% to 9%, more preferably 2% to 8%, further preferably 2.5% to 7%, more preferably 3% to 6%, most preferably 3.5%, 4%, 4.5%, 5%, or 5.5% of the total weight of the local topical preparation.

43. The local topical preparation according to claim 37, **characterized in that**, the content of microcrystalline paraffin is 2% to 50%, preferably 4% to 45%, more preferably 5% to 40%, further preferably 7% to 35%, more preferably 9% to 30%, most preferably 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 20.5%, 21% 21.5%, 22%, 22.5%, 23%, 23.5%, 24%, 24.5%, 25%, 25.5%, 26%, 26.5%, 27%, 28%, or 29% of the total weight of the local topical preparation.

44. The topical preparation according to claim 34, **characterized in that**, one or more matrices are selected from water-soluble matrices PEG 400, PEG 3350, or a combination thereof.

45. The local topical preparation according to claim 44, **characterized in that**, the content of PEG 400 is 60% to 90%, preferably 65% to 85%, most preferably 66%, 68%, 69%, 70%, 71%, 72%, 73%, 74%, 75%, 76%, 77%, 78%, 78.4%, 78.5%, 79%, 80%, 82%, or 84% of the total weight of the local topical preparation.

46. The topical preparation according to claim 44, **characterized in that**, the content of PEG 3350 is 10% to 40%, preferably 15% to 30%, most preferably 16%, 17%, 18%, 18.5%, 19%, 19.5%, 19.6%, 20%, 21%, 22%, 23%, 24%, 25%, 26%, 27%, or 28% of the total weight of the topical preparation.

47. The topical preparation according to claim 44, **characterized in that**, a content ratio of PEG 400 and PEG 3350 is 1:8-1:1, preferably 1:6-1:2, more preferably 1:5-1:3, and most preferably 1:4.

48. The topical preparation according to claim 34, **characterized in that**, the additive comprises octadecanol, stearic acid, diethylene glycol monoethyl ether, polyglycerol fatty acid ester, monoglyceryl linoleic acid, glyceryl behenate, glyceryl monostearate, or a combination of two or more of them.

49. The topical preparation according to claim 48, **characterized in that**, a content of octadecanol is 1% to 10%, preferably 2% to 9%, more preferably 3% to 8%, further preferably 4% to 7%, most preferably 4.5%, 5%, 5.5%, 6%, or 6.5% of the total weight of the topical preparation.

50. The topical preparation according to claim 48, **characterized in that**, a content of stearic acid is 1% to 10%, preferably 1.5% to 9%, more preferably 2% to 8%, further preferably 2.5% to 7%, further preferably 3% to 6%, most preferably 3.5%, 4%, 4.5%, 5%, or 5.5% of the total weight of the topical preparation.

51. The topical preparation according to claim 48, **characterized in that**, a content of diethylene glycol monoethyl ether is 1% to 10%, preferably 1.5% to 9%, more preferably 2% to 8%, further preferably 2.5% to 7%, further preferably 3% to 6%, most preferably 3.5%, 4%, 4.5%, 5%, or 5.5% of the total weight of the topical preparation.

52. The local topical preparation according to claim 48, **characterized in that**, a content of the polyglycerol fatty acid ester is 1% to 10%, preferably 1.5% to 9%, more preferably 2% to 8%, further preferably 2.5% to 7%, further preferably 3% to 6%, most preferably 3.5%, 4%, 4.5%, 5%, or 5.5% of the total weight of the local topical preparation.

53. The local topical preparation according to claim 48, **characterized in that**, a content of monolinoleic acid glyceride is 1% to 10%, preferably 1.5% to 9%, more preferably 2% to 8%, further preferably 2.5% to 7%, further preferably 3% to 6%, most preferably 3.5%, 4%, 4.5%, 5%, or 5.5% of the total weight of the local topical preparation.

54. The local topical preparation according to claim 48, **characterized in that**, the content of glyceryl behenate is 1% to 10%, preferably 2% to 9%, more preferably 3% to 8%, most preferably 3.5%, 4%, 4.5%, 5%, 5.5%, 6%, or 6.5% of the total weight of the local topical preparation.

55. The local topical preparation according to claim 48, **characterized in that**, a content of monostearic acid glycerol is 1% to 10%, preferably 2% to 9%, more preferably 3% to 8%, most preferably 3.5%, 4%, 4.5%, 5%, 5.5%, 6%, 6.5%, 7%, or 7.5% of the total weight of the local topical preparation.

56. The method for preparing a local topical preparation according to any one of claims 1-55, wherein a preparation method is selected from grinding method or melting method, preferably the melting method.

57. The topical preparation according to any one of claims 1-55, wherein the preparation is an ointment, cream, paste, gel, film, paste or patch.

58. Use of the local topical preparation according to any one of claims 1-55 in the preparation of drugs for treating skin diseases, preferably psoriasis, atopic dermatitis, vitiligo, lupus erythematosus, alopecia areata, eczema, contact dermatitis, urticaria, pompholyx, dermatomyositis, scleroderma, acne, and seborrheic dermatitis.
